# EUROPEAN PATENT APPLICATION

(11) **EP 4 079 400 A1**
(43) Date of publication of application: **26.10.2022**
(21) Application number: 21745020.4
(22) Date of filing: 22.01.2021
(51) Int. Cl.: B01J 31/04, B01J 31/02, B01J 31/12, B01J 31/14, C07B 61/00, C07C 269/06, C07C 271/18, C07C 319/20, C07C 323/41, C07K 1/00, C07K 5/06

(54) **CATALYST FOR MANUFACTURING AMIDE COMPOUND, AND METHOD FOR MANUFACTURING AMIDE COMPOUND**

(30) Priority: 22.01.2020 JP 2020008563
(71) Applicant: Chubu University Educational Foundation, Kasugai-shi Aichi 487-8501 (JP)
(72) Inventor: YAMAMOTO, Hisashi, Kasugai-shi, Aichi 487-8501 (JP); NAKASHIMA, Erika, Kasugai-shi, Aichi 487-8501 (JP)
(74) Representative: Lavoix
(86) International application number: PCT/JP2021/002306
(87) International publication number: WO 2021/149814

(57) **Abstract**

The present invention provides a catalyst containing a Brønsted acid as a novel means capable of producing an amide compound by highly stereoselectively and/or highly efficiently causing an amidation reaction in a variety of substrates having a carboxylic ester group and an amino group.

## Description

### FIELD

The present invention relates to a catalyst for manufacturing an amide compound and a method for manufacturing an amide compound.

### BACKGROUND

Conventionally, amide compounds represented by peptides have been used in a wide variety of fields, including pharmaceuticals, cosmetics, and functional foods. Development of synthetic methods thereof has been diligently pursued as an important research goal in synthetic chemistry (NPL 1 to 6). However, there are few truly effective catalysts or reactants other than carboxylic acid activators for the amidation reaction, which is the most important reaction in peptide synthesis. Therefore, it is unavoidable to use a reaction mode that forms by-products, and thus, peptide synthesis, which involves repeating multi-stage reactions, is extremely inefficient from the viewpoint of atom economy (atomic yield). The amount of by-products is large, and there are few effective purification means. As a result, the cost of disposal of by-products and purification constitutes most of the necessary costs for peptide synthesis, and is the largest obstacle to development in this field.

In peptide synthesis, which uses amino acids or derivatives thereof as starting materials, it is desirable for the amidation reaction to proceed with high stereoselectivity. Enzyme reactions in the body are examples of highly stereoselective amidation reactions. For example, in the body, peptides are synthesized with extremely high stereoselectivity through sophisticated use of enzymes and hydrogen bonds. However, enzyme reactions are not suitable for mass production, requiring enormous financial and time costs when applied to synthetic chemistry.

In synthetic chemistry, amidation reactions using catalysts have been examined, but in conventional means, the amide bond is formed primarily through the method of activating carboxylic acid, such that racemization occurs quickly, whereby synthesizing a peptide with high stereoselectivity and efficiency is difficult.

According to conventional methods, it is very difficult to link an additional amino acid or derivative to a peptide comprising a plurality of amino acids or derivatives thereof (chemical ligation) or link two or more peptides via amide bonds. As an amidation method for ligation to such peptides, there are known a method for ligation by using an amino acid having a sulfur atom to utilize the high reactivity of the sulfur atom (NPL 7) and a method for ligation by synthesizing an amino acid hydroxyamine to utilize the high reactivity of the hydroxyamine (NPL 8). However, in the former method, it is difficult to synthesize amino acids having a sulfur atom, and in the latter method, hydroxyamine synthesis involving several steps is necessary. Both methods are time-consuming and costly and have a disadvantage in efficiency.

The present inventors have developed, as techniques for synthesizing an amide compound in a highly chemoselective manner: a method of amidating a carboxylic acid/ester compound having a hydroxy group at the β-position in the presence of a specific metal catalyst (PTL 1); a method of using a hydroxyamino/imino compound as an amino acid precursor and amidating it in the presence of a specific metal catalyst, and then reducing them in the presence of a specific metal catalyst (PTL 2); and a method of amidating a carboxylic acid/ester compound in the presence of specific metal catalyst (PTL 3).

### CITATION LIST

### Patent Literature (PTL)

[PTL 1] WO 2017/204144 A
[PTL 2] WO 2018/199146 A
[PTL 3] WO 2018/199147 A

### Non-Patent Literature (NPL)

[NPL 1] Annu. Rev. Biophys. Biomol. Struct., 2005, 34, 91-118
[NPL 2] Tetrahedron, 2005, 6, 10827-10852
[NPL 3] Chem. Rev., 2007, 107, 5759-5812
[NPL 4] Chem. Rev., 2011, 111, 6557- 6602
[NPL 5] Org. Process Res. Dev., 2016, 20(2), 140-177
[NPL 6] Chem. Rev., 2016, 116, 12029-12122
[NPL 7] Science, 1992, 256, 221-225
[NPL 8] Angew. Chem. Int. Ed., 2006, 45, 1248-1252

### SUMMARY

### Problem to be Solved

However, there is still a demand to develop a new method that can produce an amide compound by generating amidation reaction efficiently and/or in a highly stereoselective manner for various reactants having a carboxylic ester group and an amino group.

Accordingly, an objective of the present invention is to provide a new method of producing an amide compound by generating amidation reaction efficiently and/or in a highly stereoselective manner for various reactants having a carboxylic ester group and an amino group.

### Means for Solving the Problem

As a result of intensive investigations, the present inventors have found that Brønsted acids have the effect of catalyzing amidation reaction between a carboxylic acid ester group and an amino group, and that the use of a Brønsted acid as a catalyst makes allows for production of an amide compound efficiently and/or in a highly stereoselective manner for various reactants having a carboxylic ester group and an amino group, thereby arriving at the present invention.

Thus, the present invention provides the following aspects.

### [Aspect 1]

A catalyst for amide reaction between a carboxylic acid ester group and an amino group, said catalyst comprising a Brønsted acid.

### [Aspect 2]

The catalyst according to Aspect 1, wherein the Brønsted acid is selected from the group consisting of compounds with pKa values of 6.0 or less.

### [Aspect 3]

The catalyst according to Aspect 1 or 2, wherein the Brønsted acid is selected from the group consisting of aliphatic or aromatic hydrocarbons having one or more carboxyl groups.

### [Aspect 4]

A method of producing, from a compound represented by general formula (1-1) and a compound represented by general formula (1-2), an amide compound represented by general formula (1-3), comprising:
causing amide reaction between the compound represented by general formula (1-1) and the compound represented by general formula (1-2) in the presence of a Brønsted acid according to any one of Aspects 1 to 3.

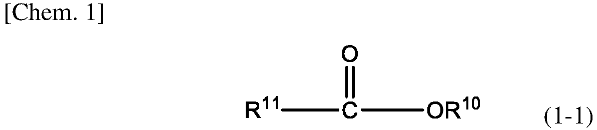

In general formula (1-1),
R¹⁰ represents a monovalent hydrocarbon group or heterocyclic group that may have one or more substituents, and
R¹¹ represents a monovalent hydrocarbon group or heterocyclic group that may have one or more substituents or a monovalent group formed by linking, optionally via a linking group, two or more multivalent hydrocarbon and/or heterocyclic groups that each may have one or more substituents.

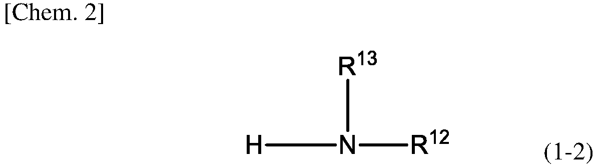

In general formula (1-2),
R¹² represents a monovalent hydrocarbon group or heterocyclic group that may have one or more substituents or a monovalent group formed by linking, optionally via a linking group, two or more multivalent hydrocarbon and/or heterocyclic groups that each may have one or more substituents, and
R¹³ represents a hydrogen atom, carboxyl group, or hydroxyl group, or a monovalent hydrocarbon group or heterocyclic group that may have one or more substituents and may be bound to the nitrogen atom via a linking group, or
R¹² and R¹³ may be bound to each other to form, together with the nitrogen atom to which R¹² and R¹³ bind, a hetero ring that may have one or more substituents.

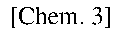

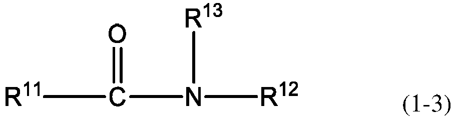

In general formula (1-3), each symbol represents the same definition as that of the same symbol in general formulae (1-1) and (1-2) above.

### [Aspect 5]

A method of producing, from a compound represented by general formula (2-1), an amide compound represented by general formula (2-2), comprising:
causing intramolecular amide reaction in the compound represented by general formula (2-1) in the presence of a Brønsted acid according to any one of Aspects 1 to 3.

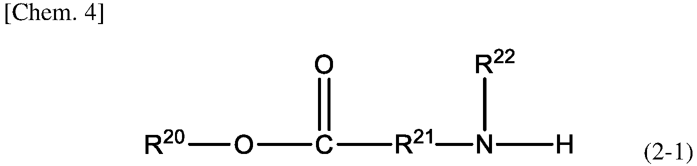

In general formula (2-1),
R²⁰ represents a monovalent hydrocarbon group or heterocyclic group that may have one or more substituents, and
R²¹ represents a divalent hydrocarbon group or heterocyclic group that may have one or more substituents or a divalent group formed by linking, optionally via a linking group, two or more multivalent hydrocarbon and/or heterocyclic groups that each may have one or more substituents, and
R²² represents a hydrogen atom, carboxyl group, or hydroxyl group, or a monovalent hydrocarbon group or heterocyclic group that may have one or more substituents and may be bound to the nitrogen atom via a linking group, or
R²¹ and R²² may be bound to each other to form, together with the nitrogen atom to which R²¹ and R²² bind, a hetero ring that may have one or more substituents.

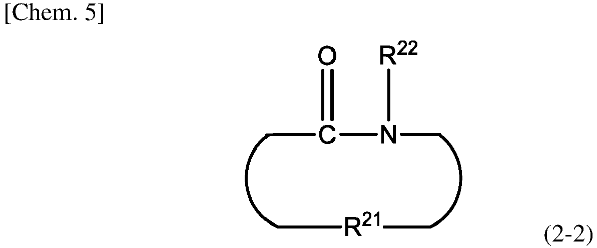

In general formula (2-2), each symbol represents the same definition as that of the same symbol in general formula (2-1) above.

### [Aspect 6]

A method of producing, from a compound represented by general formula (3-1) and a compound represented by general formula (3-2), an amide compound represented by general formula (3-3), comprising:
causing amide reaction between the compound represented by general formula (3-1) and the compound represented by general formula (3-2) in the presence of a Brønsted acid according to any one of Aspects 1 to 3.

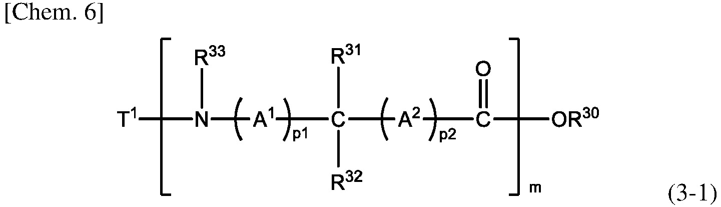

In general formula (3-1),
R³⁰ represents a monovalent hydrocarbon group or heterocyclic group that may have one or more substituents, and
R³¹ and R³², independently of each other, represent a hydrogen atom, halogen atom, hydroxyl group, carboxyl group, nitro group, cyano group, thiol group, or, a monovalent hydrocarbon group or heterocyclic group that may have one or more substituents and may be bound to the carbon atom via a linking group, and
R³³ represents a hydrogen atom, carboxyl group, hydroxyl group, or, a monovalent hydrocarbon group or heterocyclic group that may have one or more substituents and may be bound to the nitrogen atom via a linking group, or
R³¹ and R³³ may be bound to each other to form, together with the carbon atom to which R³¹ binds and the nitrogen atom to which R³³ binds, a hetero ring that may have one or more substituents,
A¹ and A², independently of each other, represent a divalent aliphatic hydrocarbon group that may have one or more substituents having 1 to 3 carbon atoms,
T¹ represents a hydrogen atom or a monovalent substituent,
p1 and p2, independently of each other, represent an integer of 0 or 1, and
m represents an integer of equal to or greater than 1 corresponding to the number of the structure units parenthesized with [ ], provided that when m is equal to or greater than 2, then the two or more structure units in [ ] may be either identical to each other or different from each other.

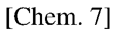

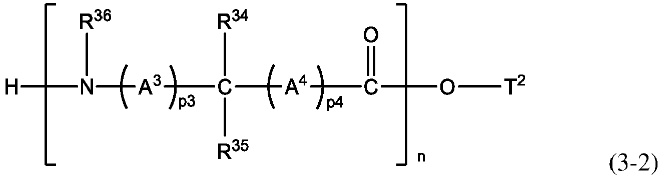

In general formula (3-2),
R³⁴and R³⁵, independently of each other, represent a hydrogen atom, halogen atom, hydroxyl group, carboxyl group, nitro group, cyano group, thiol group, or, a monovalent hydrocarbon group or heterocyclic group that may have one or more substituents and may be bound to the carbon atom via a linking group,
R³⁶ represents a hydrogen atom, carboxyl group, hydroxyl group, or, a monovalent hydrocarbon group or heterocyclic group that may have one or more substituents and may be bound to the nitrogen atom via a linking group,
R³⁴ and R³⁶ may be bound to each other to form, together with the carbon atom to which R³⁴ binds and the nitrogen atom to which R³⁶ binds, a hetero ring that may have one or more substituents,
A³and A⁴, independently of each other, represent a divalent aliphatic hydrocarbon group that may have one or more substituents having 1 to 3 carbon atoms,
T² represents a hydrogen atom or a monovalent substituent,
p3 and p4, independently of each other, represent an integer of 0 or 1, and
n represents an integer of equal to or greater than 1 corresponding to the number of the structure units parenthesized with [ ], provided that when n is equal to or greater than 2, then the two or more structure units in [ ] may be either identical to each other or different from each other.

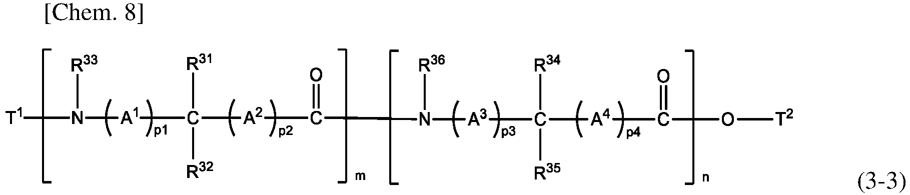

In general formula (3-3), each symbol represents the same definition as that of the same symbol in general formulae (3-1) and (3-2) above.

### [Aspect 7]

The method according to any one of Aspects 4 to 6, wherein R¹⁰ in general formula (1-1), R²⁰ in general formula (2-1), or R³⁰ in general formula (3-1) is a monovalent aromatic group or heterocyclic group which may have one or more substituents.

### [Aspect 8]

The method according to any one of Aspects 3 to 7, wherein the reaction is carried out as a batch reaction or a flow reaction.

### EFFECTS

The method according to the present invention allows for the production of an amide compound by using a Brønsted acid as a catalyst to cause an amidation reaction efficiently and/or in a highly stereoselective manner for various reactants having a carboxylic ester group and an amino group.

### EMBODIMENTS

The present invention is described hereinafter in detail with reference to specific embodiments thereof. However, the present invention is not limited to the following embodiments and can be carried out in any embodiment that does not deviate from the gist of the present invention.

All the patent publication, unexamined patent publications, and non-patent literature cited in the present disclosure are incorporated herein by reference in their entireties for any purpose.

In the following description, the reactant compound having a carboxylic ester group subject to amidation reaction may be referred to as "Reactant Compound 1," and the reactant compound having an amino group subject to amidation reaction as "Reactant Compound 2." When the amidation reaction between the carboxylic ester group and the amino group in the same reactant compound is catalyzed (i.e., in the case of mode (2) described below), Reactant Compound 1 and Reactant Compound 2 refer to the same compound.

### ^{∗}Definition of Terms:

The term "amino acid" herein refers to a compound having a carboxyl group and an amino group. Unless otherwise specified, the type of an amino acid is not particularly limited. For example, from the viewpoint of optical isomerism, an amino acid may be a D-amino acid or an L-amino acid. From the viewpoint of the relative positions of the carboxyl group and the amino group, an amino acid may be any of an α-amino acid, β-amino acid, γ-amino acid, δ-amino acid, or ε-amino acid. Examples of amino acids include, but are not limited to, natural amino acids that make up proteins. Examples include valine, leucine, isoleucine, alanine, arginine, glutamine, lysine, aspartic acid, glutamic acid, proline, cysteine, threonine, methionine, histidine, phenylalanine, tyrosine, tryptophan, asparagine, glycine, and serine.

The term "peptide" herein refers to a compound comprising a plurality of amino acids linked together via peptide bonds. Unless otherwise specified, the plurality of amino acid units constituting a peptide may be the same type of amino acid unit or may consist of two or more types of amino acid units. The number of amino acids constituting a peptide is not restricted as long as it is two or more. Examples include 2 (also called "dipeptide"), 3 (also called "tripeptide"), 4 (also called "tetrapeptide"), 5 (also called "pentapeptide"), 6, 7, 8, 9, 10, 15, 20, 30, 40, 50, 100, or more.

The term "lactam" herein refers to a compound comprising a ring formed by an intramolecular amidation bond between a carboxyl group and an amino group in a single molecule, and the term "cyclic peptide" herein refers to a peptide comprising a ring formed by an intramolecular amidation bond between a carboxyl group and an amino group (for example, but not limited to, between a terminal carboxyl group and a terminal amino group) in a single peptide molecule.

The term "amino group" herein refers to a functional group represented by any formula of -NH2, -NRH, and -NRR' (where R and R' each represent a substituent) obtained by removing hydrogen from ammonia, a primary amine, and a secondary amine, respectively.

Unless otherwise specified, a hydrocarbon group herein may be either aliphatic or aromatic. An aliphatic hydrocarbon group may be in the form of either a chain or a ring. A chain hydrocarbon group may be linear or branched. A cyclic hydrocarbon group may be monocyclic, bridged cyclic, or spirocyclic. The hydrocarbon group may be saturated or unsaturated. In other words, one, two, or more carbon-carbon double and/or triple bonds may be included. Specifically, "hydrocarbon group" represents a concept including an alkyl group, alkenyl group, alkynyl group, cycloalkyl group, cycloalkenyl group, cycloalkynyl group, aryl group, etc. Unless otherwise specified, one, two, or more hydrogen atoms of the hydrocarbon group may be replaced with any substituents and one, two, or more carbon atoms of the hydrocarbon group may be replaced with any heteroatoms corresponding to the valence thereof.

The term "hydrocarbon oxy group" herein refers to a group comprising an oxy group (-O-) linked via one bond thereof to the hydrocarbon group as defined above.

The term "hydrocarbon carbonyl group" herein refers to a group comprising a carbonyl group (-C(=O)-) linked via one bond thereof to the hydrocarbon group as defined above.

The term "hydrocarbon sulfonyl group" herein refers to a group comprising a sulfonyl group (-S(=O)₂-) linked via one bond thereof to the hydrocarbon group as defined above.

A heterocyclic group may be saturated or unsaturated. In other words, it may contain one, two, or more carbon-carbon double and/or triple bonds. A heterocyclic group may be monocyclic, bridged cyclic, or spirocyclic. The heteroatom included in the constituent atoms of the heterocyclic group is not particularly limited, examples thereof including nitrogen, oxygen, sulfur, phosphorus, and silicon.

The term "heterocyclic oxy group" herein refers to a group comprising an oxy group (-O-) linked via one bond thereof to the heterocyclic group as defined above.

The term "heterocyclic carbonyl group" herein refers to a group comprising a carbonyl group (-C(=O)-) linked via one bond thereof to the heterocyclic group as defined above.

The term "heterocyclic sulfonyl group" herein refers to a group comprising a sulfonyl group (-S(=O)₂-) linked via one bond thereof to the heterocyclic group as defined above.

Unless otherwise specified, the term "substituent" herein refers, independently of each other, to any substituent which is not particularly limited so long as the amidation step of the production method according to the present invention proceeds. Examples include, but are not limited to, a halogen atom, hydroxyl group, carboxyl group, nitro group, cyano group, thiol group, sulfonic acid group, amino group, amide group, imino group, imide group, hydrocarbon group, heterocyclic group, hydrocarbon oxy group, hydrocarbon carbonyl group (acyl group), hydrocarbon oxycarbonyl group, hydrocarbon carbonyl oxy group, hydrocarbon substitution amino group, hydrocarbon substitution amino carbonyl group, hydrocarbon carbonyl substitution amino group, hydrocarbon substitution thiol group, hydrocarbon sulfonyl group, hydrocarbon oxysulfonyl group, hydrocarbon sulfonyl oxy group, heterocyclic oxy group, heterocyclic carbonyl group, heterocyclic oxycarbonyl group, heterocyclic carbonyl oxy group, heterocyclic amino group, heterocyclic amino carbonyl group, heterocyclic carbonyl substitution amino group, heterocyclic substitution thiol group, heterocyclic sulfonyl group, heterocyclic oxysulfonyl group, and heterocyclic sulfonyl oxy group. The term "substituents" also may include functional groups obtained by substituting any of the aforementioned functional groups with any of the aforementioned functional groups as long as the valence and physical properties thereof permit. When a functional group has one or more substituents, the number of the substituents is not particularly limited as long as the valence and physical properties thereof permit. When the functional group has two or more substituents, they may be identical to each other or different from each other.

The term Me herein refers to a methyl group, the term Et herein refers to a ethyl group, the term Pr herein refers to a propyl group, the term i-Pr herein refers to an isopropyl group, the term Bu herein refers to a butyl group, and the term t-Bu herein refers to a Tert-butyl group.

The term Ac herein refers to a acetyl group, the term acac herein refers to an acetylacetonate, the term Cp herein refers to a cyclopentadienyl, the term Tf herein refers to a trifluoromethanesulfonyl, the term Trt herein refers to a trityl group, the term THF herein refers to a tetrahydrofuran, the term DCM herein refers to a dichloromethane, and the term DMSO herein refers to a dimethyl sulfoxide.

Amino acids and residues thereof may herein be represented by three-letter abbreviations well known to a person skilled in the art. The three-letter abbreviations of major amino acids are shown in the following table.

**[Table 1]**

| | |
|---|---|
| Ala | Alanine |
| Arg | Arginine |
| Asn | Asparagine |
| Asp | Aspartic acid |
| Cys | Cysteine |
| Gln | Glutamine |
| Glu | glutamic acid |
| Gly | Glycine |
| His | Histidine |
| Ile | Isoleucine |
| Leu | Leucine |
| Lys | Lysine |
| Met | Methionine |
| Phe | Phenylalanine |
| Phg | Phenylglycine |
| Pro | Proline |
| Ser | Serine |
| Thr | Threonine |
| Trp | Tryptophan |
| Tyr | Tyrosine |
| Val | Valine |

β-homoamino acids and residues thereof may herein be represented by "Ho" followed by three-letter abbreviations of corresponding β-amino acids.

The term "reaction agent" for an amide reaction between a carboxyl group and an amino group herein refers to an agent capable of causing or promoting an amide reaction between a carboxyl group and an amino group.

### ^{∗}Catalyst of the Present Invention:

One aspect of the present invention relates to a catalyst containing a Brønsted acid for the amide reaction of an ester group with an amino group (hereinafter also referred to as "the catalyst of the present invention").

The term "Brønsted acid" herein refers to an acid according to the Brønsted-Lawry theory. According to the Brønsted-Lowry theory, an acid is defined as a compound that functions as a proton (hydrogen ion) donor and a base as a compound that functions as a proton acceptor. In other words, the Brønsted acid to be contained in the catalyst of the present invention refers to a compound that functions as a proton donor.

The Brønsted acid to be contained in the catalyst of the present invention is not particularly limited, and may be any compound so long as it functions as a proton donor. However, the Brønsted acid in the catalyst of the present invention may preferably be a compound that satisfies at least any one of the following features.

From the viewpoint of ensuring its function as a proton donor, the Brønsted acid in the catalyst of the present invention may preferably have a pKa value of below a predetermined value. Specifically, the pKa value may preferably be 6.0 or less, particularly 5.5 or less.

The Brønsted acid in the catalyst of the present invention may also preferably be an aliphatic or aromatic hydrocarbon compound having one or two carboxyl groups, i.e., a carboxylic acid compound. In this case, from the viewpoint of preventing a decrease in the acidity of the carboxylic acid compound, it may be preferred that the carbon atom corresponding to the alpha position of each carboxyl group in the carboxylic acid compound does not have an electron-withdrawing substituent. In addition, from the viewpoint of preventing amidation of the Brønsted acid itself, it may also be preferred that the aliphatic or aromatic hydrocarbon group to which the carboxyl group is attached has a large molecular weight (e.g., 3 or more carbons, particularly 4 or more carbons, more particularly 5 or more carbons) and tends to cause steric hindrance.

However, Brønsted acids having the physical properties, characteristics, and/or structures described above are merely examples of Brønsted acids preferred for use in the catalyst according to the present invention. Some Brønsted acids not having these physical properties, characteristics, and/or structures may exhibit high amidation activity, as demonstrated in the Examples below, and may preferably be used in the catalyst according to the present invention.

Specific examples of Brønsted acids for use in the catalyst according to the present invention include 2,6-di-tert-butylbenzoic acid, pivalic acid, isobutyric acid, diisopropylacetic acid, valeric acid, isophthalic acid, 2,4,6-trimethylbenzoic acid, propionic acid, adipic acid, acetic acid, butanoic acid, 2-tert-butyl-3,3-dimethylbutanoic acid, 2,6-diisopropylbenzoic acid, 2,4,6-triisopropylbenzoic acid, caproic acid, thioacetic acid, succinic acid, ammonium acetate, 2,6-dimethylbenzoic acid, benzoic acid, malonic acid, 1-adamantane carboxylic acid, 4-nitrophenol, (S)-2-(1H-tetrazol-5-yl)pyrrolidine-1-carboxylic acid, tert-butyl ester, maleic acid, trichlorolic acid, 2,6-dimethoxy benzoic acid, lactic acid, bromoacetic acid, trichloroacetic acid, catechol, 1,2-phenylene diacetate, trifluoroacetic acid, formic acid, phthalic acid, 1,1'-binaphthyl-2,2'-dicarboxylic acid, (+)-10-camphorsulfonic acid, methanesulfonic acid, lactic acid methyl, benzenethiol, N-(2,6-dimethylphenyl)-1,1,1-trifluoromethanesulfonamide, L-malic acid, N-[2,6-bis(1-methylethyl)phenyl] -1,1,1-trifluoromethanesulfonamide, 4-methylphenylsulfonic acid anhydride, 4-methoxyphenylboronic acid, 2,2,2-trifluoroethanol, fumaric acid, ethyl lactate, L-proline, phenol, 4-methoxyphenol, p-toluene boronic acid, oxalic acid, proline tetrazole, triphenylsilanol, citric acid, 2,4,6-trimethylphenol, 2,6-di-tert-butyl-4-methylphenol, L-tartaric acid, tert-butyldimethylsilanol, and pyromellitic acid.

Preferred examples of Brønsted acids for use in the catalyst of the present invention include 2,6-di-tert-butylbenzoic acid, pivalic acid, isobutyric acid, diisopropylacetic acid, valeric acid, isophthalic acid, 2,4,6-trimethylbenzoic acid, propionic acid, adipic acid, acetic acid, butanoic acid, 2-tert-butyl-3,3-dimethylbutanoic acid, 2,6-diisopropylbenzoic acid, 2,4,6-triisopropylbenzoic acid, caproic acid, thioacetic acid, succinic acid, ammonium acetate, 2,6-dimethylbenzoic acid, benzoic acid, malonic acid, 1-adamantane carboxylic acid, 4-nitrophenol, (S)-2-(1H-tetrazol-5-yl)pyrrolidine-1-carboxylic acid, tert-butyl ester, maleic acid, trichlorolic acid, 2,6-dimethoxy benzoic acid, lactic acid, and bromoacetic acid. More preferred among them are 2,6-di-tert-butylbenzoic acid, pivalic acid, isobutyric acid, diisopropylacetic acid, valeric acid, isophthalic acid, 2,4,6-trimethylbenzoic acid, propionic acid, adipic acid, acetic acid, butanoic acid, 2-tert-butyl-3,3-dimethylbutanoic acid, 2,6-diisopropylbenzoic acid, 2,4,6-triisopropylbenzoic acid, caproic acid, thioacetic acid, succinic acid, ammonium acetate, and 2,6-dimethylbenzoic acid, especially preferred being 2,6-di-tert-butylbenzoic acid, pivalic acid, isobutyric acid, diisopropylacetic acid, valeric acid, isophthalic acid, 2,4,6-trimethylbenzoic acid, propionic acid, and adipic acid.

These Brønsted acids may be used in the catalyst of the present invention either by selecting any one compound singly or selecting any two or more compounds in any combination at any ratios.

The catalyst of the present invention using Brønsted acid makes it possible to produce amide compounds by generating amidation for various reactants having carboxylic ester groups and amino groups reaction efficiently and/or in a highly stereoselective manner. The catalyst of the present invention also makes the isolation and purification of the products after the reaction much easier, since it eliminates the need for metal catalysts and other additives which are necessary in conventional amidation methods, and also eliminates the need for solvents (reaction medium) depending on the reactant compounds and the reaction conditions.

The reason why the catalyst of the present invention using Brønsted acid enables such highly efficient and/or stereoselective amidation reactions is not certain. However, without being bound by any theory, it is presumed that the Brønsted acid in the catalyst of the present invention functions as a proton donor and catalyzes the amide reaction between the carboxylic ester group of Reactant Compound 1 and the amino group of Reactant Compound 2. It is also presumed that in the case of Brønsted acids having at least one carboxyl group (i.e., carboxylic acids) in particular, the carboxyl group of the Brønsted acid forms carboxylic acid dimers with the carboxylic ester group of Reactant Compound 1 and activates the carboxyl group of Reactant Compound 1 to thereby catalyze amide reaction with the amino group of Reactant Compound 2.

### ^{∗}Production method according to the present invention :

Another aspect of the present invention relates to a method of producing an amide compound method (hereinafter also referred to as "the production method according to the present invention") using the Brønsted acid as a reaction agent. The method includes using one or more compounds having a carboxyl ester group and/or an amino group as reactants, and causing an amidation reaction between the carboxyl ester group and the amino group. The production method of the present invention can be broadly classified into the following modes, depending on the types of the reactants used and the target compound produced by the amidation reaction.

(1) A method including using a first compound having a carboxyl ester group (Reactant Compound 1) and a second compound having an amino group (Reactant Compound 2) as reactants and producing a third compound (amide compound) in the presence of the Brønsted acid described above, by causing an intermolecular amidation reaction between the carboxyl ester group of the first compound and the amino group of the second compound, thereby linking these compounds with an amide bond (hereinafter also referred to as "Embodiment (1)").
(2) A method including using a first compound having a carboxyl ester group and an amino group (Reactant Compound 1 and 2) as a reactant and producing a second compound (lactam compound) in the presence of the Brønsted acid described above, by causing an intramolecular amidation reaction (lactamization reaction) between the carboxyl ester group and the amino group of the first compound, thereby cyclizing the first compound with an amide bond (hereinafter also referred to as "Embodiment (2)").

In addition, among specific embodiments included in Embodiment (1) above, the following specific embodiment is especially interesting.

(3) A method including using an amino acid or a peptide as each of the first compound having a carboxyl ester group (Reactant Compound 1) and the second compound having an amino group (Reactant Compound 2) as reactants and producing a peptide compound as the third compound in the presence of the Brønsted acid described above, by causing an intermolecular amidation reaction between the carboxyl ester group of the first compound and the amino group of the second compound, thereby linking these compounds with an amide bond (hereinafter also referred to as "Embodiment (3)").

Likewise, Embodiment (2) includes a specific embodiment in which the first compound (Reactant Compound 1 and 2) is a peptide and the second compound (lactam compound) is a cyclic peptide.

The combinations of the reactants and the target compounds in Embodiments (1) to (3) are described below.

### ^{∗}Embodiment (1):

Embodiment (1) relates to a method for using a compound having a carboxyl ester group represented by formula (1-1) (hereinafter also referred to as "compound (1-1)") and a compound having an amino group represented by formula (1-2) (hereinafter also referred to as "compound (1-2)") as reactants, and producing an amide compound represented by formula (1-3) by causing an intermolecular amidation reaction between the carboxyl ester group of compound (1-1) and the amino group of compound (1-2) in the presence of a Brønsted acid as described above mentioned above.

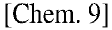

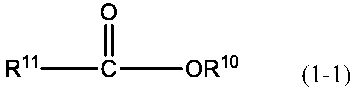

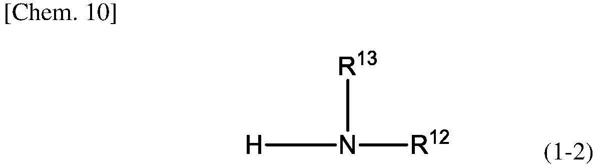

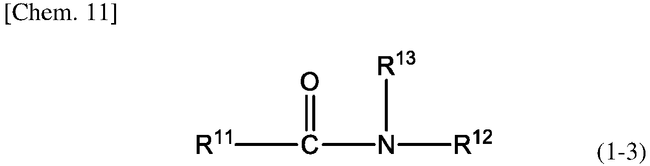

In other words, in this embodiment, the carboxyl ester group and the amino group possessed by the different compounds (1-1) and (1-2) are linked via an amidation reaction to produce a novel amide compound (1-3).

The definition of each symbol in general formulae (1-1), (1-2), and (1-3) are as follows.

R¹⁰ represents a monovalent hydrocarbon group or heterocyclic group that may have one or more substituents.

R¹¹and R¹², independently of each other, represent (i) a monovalent hydrocarbon group or heterocyclic group that may have one or more substituents or (ii) a monovalent group formed by linking, optionally via a linking group, two or more multivalent hydrocarbon and/or heterocyclic groups that each may have one or more substituents. Roughly speaking, when each of R¹¹ and/or R¹² is (i), then the compound (1-1) and/or (1-2) may be a monomer or a low molecular compound, while when each of R¹¹ and/or R¹² is (ii), then the compound (1-1) and/or (1-2) may be a polymer or a macromolecular compound.

When each of R¹¹and R¹² is, independently of each other, (i) a monovalent hydrocarbon group or heterocyclic group that may have one or more substituents, the details thereof are as follows.

When each of R¹¹and/or R¹² is a monovalent hydrocarbon group that may have one or more substituents, the number of carbon atoms of the hydrocarbon group (including its substituents, if any) is not particularly limited, but the upper limit thereof may be, for example, 40 or less, 30 or less, 20 or less, 16 or less, or 12 or less. The lower limit thereof depends on the type of the hydrocarbon group, but may be 1 or more for alkyl groups, 2 or more for alkenyl and alkynyl groups, and 3 or more, such as 4 or more, or 5 or more, for cycloalkyl groups. Examples of the number of atoms include, for example, 1, 2, 3, 4, 5, 6, 7, 8, 9, 10, 11, 12, 13, 14, 15, 16, 17, 18, 19, 20, 22, 24, 26, 28, 30, 32, 34, 36, 38, or 40.

When each of R¹¹and/or R¹² is a monovalent heterocyclic group that may have one or more substituents, the total number of carbon atoms and hetero atoms of the heterocyclic group (including its substituents, if any) is not particularly limited, but the upper limit thereof may be, for example, 20 or less, 15 or less, 10 or less, 8 or less, or 6 or less. The lower limit thereof depends on the type of the heterocyclic structure, but may typically be 3 or more, for example 4 or more, or 5 or more. Examples of the number of atoms include, for example, 3, 4, 5, 6, 7, 8, 9, 10, 11, 12, 13, 14, 15, 16, 17, 18, 19, 20, 22, 24, 26, 28, 30, 32, 34, 36, 38, or 40.

Among them, each of R¹¹and R¹² may preferably be, independently of each other, an amino group, alkyl group, alkenyl group, cycloalkyl group, alkoxy group, aryl group, aryloxy group, acyl group, heterocyclic group, or heterocyclic oxy group that may have one or more substituents.

Examples of R¹¹and R¹² include, but are not limited to, the following.
^{∗}Alkyl groups such as methyl group, ethyl group, propyl group, isopropyl group, n-butyl group, isobutyl group, tert-butyl group, sec-butyl group, pentyl group, isopentyl group, neopentyl group, hexyl group, heptyl group, octyl group, decyl group, and nonyl group; ^{∗}Cycloalkyl groups such as cyclopropyl group, cyclobutyl group, cyclopentyl group, cyclohexyl group, cycloheptyl group, bicyclooctyl group, and spirooctyl group; ^{∗}Aryl groups such as phenyl group, benzyl group, tolyl group, naphthyl group, and anthracenyl group;
^{∗}Heterocyclic groups such as furanyl group, thiophenyl group, pyranyl group, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, 2,5-dihydro-1,3-thiazolyl group, and carbazolyl group; and ^{∗}Heterocyclic oxy groups such as furanyl oxy group, pyrrolyl oxy group, indolyl oxy group, and quinolyl oxy group.

Of the groups mentioned above, those having a carboxyl group may or may not have a protective group. When such a group with a carboxyl group has a protective group, the reaction selectivity with the carboxyl group on the right side of formula (1-1) is usually superior to that with the carboxyl groups present on the other substituents, although it may also depend on the reactivity of the compound (1-1) and the compound (1-2) used in the reaction.

When each of R¹¹and R¹² is, independently of each other, (ii) a monovalent group formed by linking, optionally via a linking group, two or more multivalent hydrocarbon and/or heterocyclic groups that each may have one or more substituents, the details thereof are as follows.

Such multivalent hydrocarbon groups or heterocyclic groups include multivalent (e.g., di-, tri-, tetra-, or penta-valent, or even higher) hydrocarbon groups or heterocyclic groups obtained by removing one or more hydrogen atoms from the monovalent hydrocarbon or heterocyclic groups mentioned above. When two or more (e.g., two, three, four, five, or more) of these multivalent hydrocarbon or heterocyclic groups are linked together, they may be linked by direct bonds or with intervening linking groups. Such linking groups are not particularly limited, but may be selected, independently of each other, from the structures listed below (where, in the chemical formulae below, A represents a monovalent hydrocarbon group or a heterocyclic group, each of which may independently have one or more substituents. When two A's are present in the same group, they may be identical to each other or different from each other.).

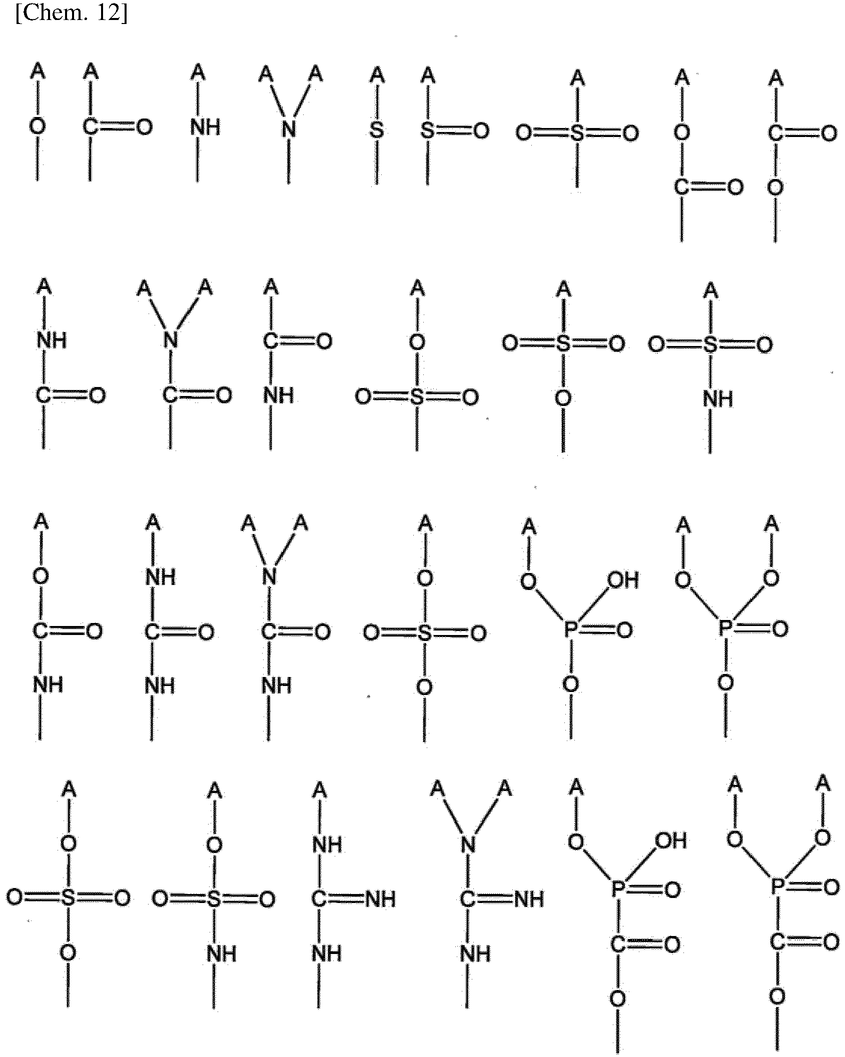

As mentioned above, when each of R¹¹and/or R¹² is (ii) a monovalent group formed by linking, optionally via a linking group, two or more multivalent hydrocarbon and/or heterocyclic groups that each may have one or more substituents, then the compound (1-1) and/or (1-2) typically is a polymer or a macromolecular compound. Examples of such polymers and macromolecular compounds include, but are not limited to, the following.

^{∗}Peptides/proteins (which will be explained in Embodiment (3) below in details);
^{∗}Nucleic acids (e.g., DNA (deoxyribonucleic acid) and RNA (ribonucleic acid));
^{∗}Polysaccharides (e.g., cellulose, amylose, starch, chitin, and chitosan);
^{∗}Complex polysaccharides (e.g., lipopolysaccharides and glycoproteins);
^{∗}Lipids (e.g., simple lipids, phospholipids, and glycolipids);
^{∗}Natural and synthetic resins (e.g., phenol resins, silicone resins, epoxy resins, melamine resins, urea resins, unsaturated polyester resins, alkyd resins, polyvinyl chloride, polyethylene, polyethylene glycol, polypropylene, polystyrene, polyvinyl acetate, polylactate, polyester, polyurethane, polyamide (e.g., nylon), (meth)acrylic resin, polyvinyl chloride, polyvinylidene chloride, and natural and synthetic rubbers).

R¹³ represents a hydrogen atom, carboxyl group, or hydroxyl group, or a monovalent hydrocarbon group or heterocyclic group that may have one or more substituents. The details of the substituents, if any, are described above. Examples of the number of the substituents include 5, 4, 3, 2, 1, or 0.

When R¹³ is a monovalent hydrocarbon group or heterocyclic group that may have one or more substituents, there may be an intervening linking group between the hydrocarbon group or heterocyclic group and the nitrogen atom to which the hydrocarbon group or heterocyclic group binds. Such linking groups are not particularly limited, but may be selected, independently of each other, from the structures shown below (where, in the chemical formulae below, A represents a monovalent hydrocarbon group or a heterocyclic group, each of which may independently have one or more substituents. When two A's are present in the same group, they may be identical to each other or different from each other).

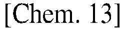

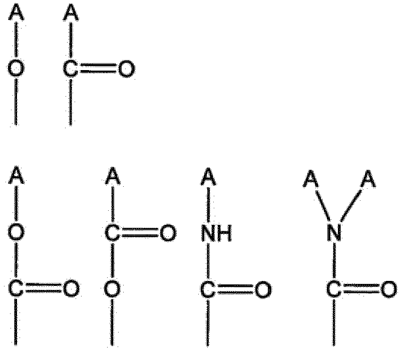

The upper limit of the number of the carbon atoms contained in each hydrocarbon group (including its substituents, if any) may be, for example, 20 or less, 15 or less, 10 or less, 8 or less, or 6 or less. The lower limit thereof depends on the type of the hydrocarbon group, but may be 1 or more for alkyl groups, 2 or more for alkenyl and alkynyl groups, and 3 or more, such as 4 or more, or 5 or more, for cycloalkyl groups. Examples of the number of atoms include, for example, 1, 2, 3, 4, 5, 6, 7, 8, 9, 10, 11, 12, 13, 14, 15, 16, 17, 18, 19, or 20.

The upper limit of the number of the carbon atoms and hetero atoms included in each heterocyclic group (including its substituents, if any) may be, for example, 20 or less, 15 or less, 10 or less, 8 or less, or 6 or less. The lower limit thereof depends on the type of the heterocyclic structure, but may be typically 3 or more, for example 4 or more, or 5 or more. Examples of the number of atoms include, for example, 3, 4, 5, 6, 7, 8, 9, 10, 11, 12, 13, 14, 15, 16, 17, 18, 19, or 20.

R¹² and R¹³ may be bound to each other to form, together with the nitrogen atom to which R¹² and R¹³ bind, a hetero ring that may have one or more substituents. The details of the substituents, if any, are described above. Examples of the number of such substituents include, for example, 5, 4, 3, 2, 1, or 0.

The upper limit of the total number of carbon atoms and hetero atoms included in each heterocyclic group (including its substituents, if any) may be, for example 20 or less, 15 or less, 10 or less, 8 or less, or 6 or less. The lower limit thereof depends on the type of the heterocyclic structure, but may be typically 3 or more, for example 4 or more, or 5 or more. Examples of the number of atoms include, for example, 3, 4, 5, 6, 7, 8, 9, 10, 11, 12, 13, 14, 15, 16, 17, 18, 19, or 20.

Examples of such hetero rings include, but are not limited to, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, and 2,5-dihydro-1,3-thiazolyl group.

### ^{∗}Embodiment (2):

Embodiment (1) relates to a method for using a compound having a carboxyl ester group and an amino group represented by formula (2-1) (hereinafter also referred to as "compound (2-1)") as a reactant, and producing a lactam compound represented by formula (2-2) by causing an intramolecular amidation reaction between the carboxyl ester group and the amino group of compound (2-1) in the presence of a Brønsted acid as described above mentioned above.

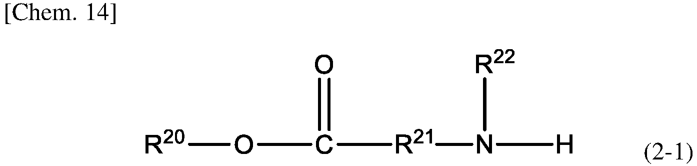

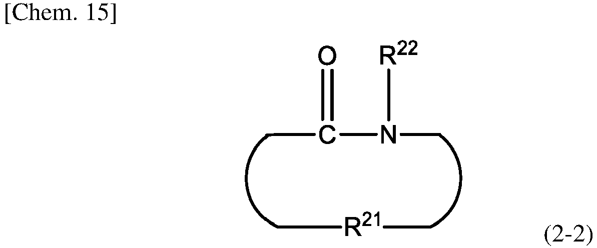

In other words, in this embodiment, the carboxyl ester group and the amino group possessed by the same compound (2-1) are linked via an intramolecular amidation reaction to cause cyclization and produce a novel lactam compound (2-2).

The definition of each symbol in general formulae (2-1) and (2-2) are as follows.

R²⁰ represents a monovalent hydrocarbon group or heterocyclic group that may have one or more substituents. The details are the same as those described earlier for R¹⁰ of formula (1-1) above.

R²¹ represents (i) a divalent hydrocarbon group or heterocyclic group that may have one or more substituents or (ii) a divalent group formed by linking, optionally via a linking group, two or more multivalent hydrocarbon and/or heterocyclic groups that each may have one or more substituents. Roughly speaking, when R²¹ is (i), then the compound (2-1) may be a monomer or a low molecular compound, while when R²¹ is (ii), then the compound (2-1) may be a polymer or a macromolecular compound.

When R²¹ is (i) a divalent hydrocarbon group or heterocyclic group that may have one or more substituents, examples thereof include divalent hydrocarbon or heterocyclic groups obtained by removing any hydrogen atom from the monovalent hydrocarbon or heterocyclic groups described for R¹¹ in formula (1-1) and R¹² in formula (1-2) above. Other details are the same as those described for R¹¹ in formula (1-1) and R¹² in formula (1-2) above.

On the other hand, when R²¹ is (ii) a divalent group formed by linking, optionally via a linking group, two or more multivalent hydrocarbon and/or heterocyclic groups that each may have one or more substituents, examples thereof include divalent groups obtained by removing any hydrogen atom from the monovalent groups including two or more multivalent hydrocarbon groups or heterocyclic groups linked together described for R¹¹ in formula (1-1) and R¹² in formula (1-2) above. Other details are the same as those described for R¹¹ in formula (1-1) and R¹² in formula (1-2) above.

R²² represents a hydrogen atom, carboxyl group, hydroxyl group, or a monovalent hydrocarbon group or heterocyclic group that may have one or more substituents. Details of R²² are the same as those described for R¹³ in formula (1-2) above.

R²¹ and R²² may be bound to each other to form, together with the nitrogen atom to which R²¹ and R²² bind, a hetero ring that may have one or more substituents. The details of such a hetero ring are the same as those described for the hetero ring formed by the bonding of R¹² and R¹³ in formula (1-2) above.

### ^{∗}Embodiment (3):

Embodiment (3) relates to a method for using an amino acid or peptide represented by formula (3-1) (hereinafter also referred to as "compound (3-1)") and an amino acid or peptide represented by formula (3-2) (hereinafter also referred to as "compound (3-2)") as reactants, and producing a peptide represented by formula (3-3) by causing an intermolecular amidation reaction between the terminal carboxyl ester group of compound (3-1) and the terminal amino group of compound (3-2) in the presence of a Brønsted acid as described above mentioned above.

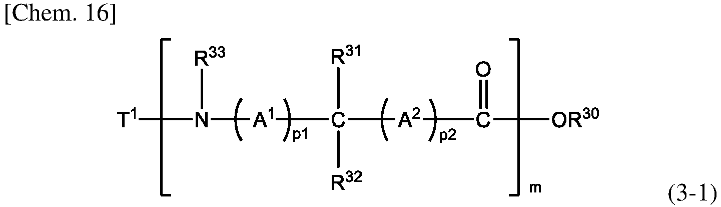

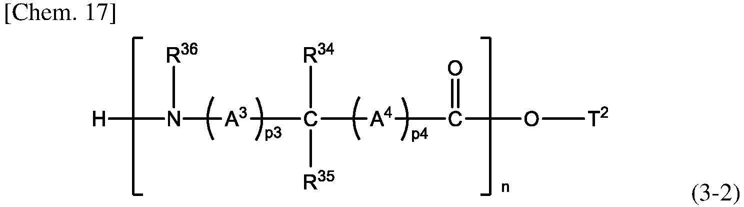

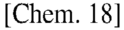

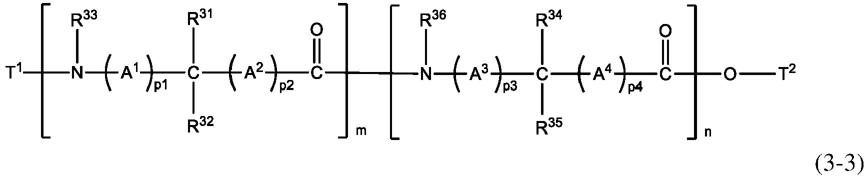

In other words, in this embodiment, the carboxyl and amino groups possessed by the different peptides (3-1) and (3-2) are linked via an amidation reaction to produce a novel peptide (3-3).

The definition of each symbol in general formulae (3-1), (3-2), and (3-3) are as follows.

R³⁰ represents a monovalent hydrocarbon group or heterocyclic group that may have one or more substituents. The details are the same as those described earlier for R¹⁰ and R¹¹ of formula (1-1) above.

R³¹, R³², R³⁴, and R³⁶, independently of each other, represent a hydrogen atom, halogen atom, hydroxyl group, carboxyl group, nitro group, cyano group, or thiol group, or a monovalent hydrocarbon group or heterocyclic group that may have one or more substituents. The details of the substituents, if any, are described above. Examples of the number of such substituents include, for example, 5, 4, 3, 2, 1, or 0.

When each of R³¹, R³², R³⁴, and/or R³⁶ is a monovalent hydrocarbon group or heterocyclic group that may have one or more substituents, then there may be an intervening linking group between the hydrocarbon group or heterocyclic group and the carbon atom to which the hydrocarbon group or heterocyclic group binds. Such linking groups are not limited, but may be selected, independently of each other, from the structures indicated below (where, in the chemical formulae below, A represents a monovalent hydrocarbon group or a heterocyclic group, each of which may independently have one or more substituents. When two A's are present in the same group, they may be identical to each other or different from each other).

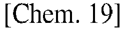

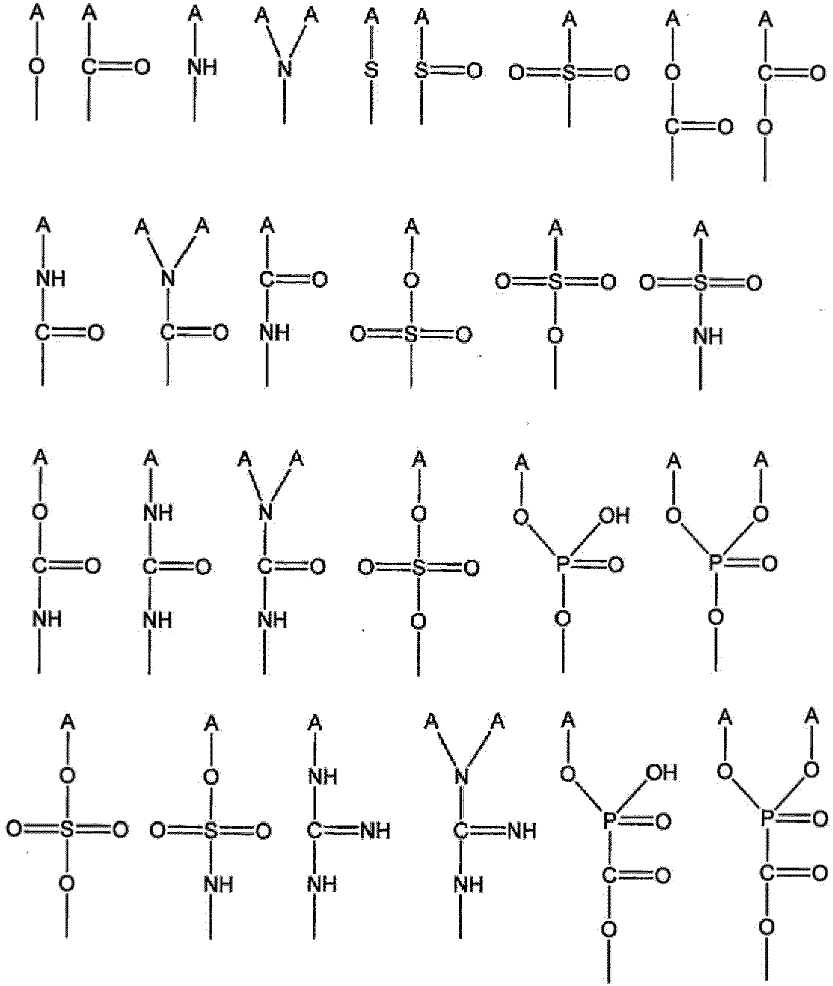

The number of carbon atoms contained in each hydrocarbon group (including its substituents, if any) is not particularly limited, but the upper limit thereof may be, for example, 20 or less, 15 or less, 10 or less, 8 or less, or 6 or less. The lower limit thereof depends on the type of the hydrocarbon group, but may be 1 or more for alkyl groups, 2 or more for alkenyl and alkynyl groups, and 3 or more, such as 4 or more, or 5 or more, for cycloalkyl groups. Examples of the number of atoms include, for example, 1, 2, 3, 4, 5, 6, 7, 8, 9, 10, 11, 12, 13, 14, 15, 16, 17, 18, 19, or 20.

The upper limit of the total number of carbon atoms and hetero atoms included in each heterocyclic group (including its substituents, if any) may be, for example 20 or less, 15 or less, 10 or less, 8 or less, or 6 or less. The lower limit thereof depends on the type of the heterocyclic structure, but may be typically 3 or more, for example 4 or more, or 5 or more. Examples of the number of atoms include, for example, 3, 4, 5, 6, 7, 8, 9, 10, 11, 12, 13, 14, 15, 16, 17, 18, 19, or 20.

Among them, each of R³¹, R³², R³⁴, and R³⁶ may preferably be selected, independently of each other, from hydrogen atom, hydroxyl group, thiol group, carboxyl group, nitro group, cyano group, or halogen atom, or, that may have one or more substituents, amino group, alkyl group, alkenyl group, cycloalkyl group, alkoxy group, aryl group, aryloxy group, acyl group, heterocyclic group, and heterocyclic oxy group.

Examples of R³¹, R³², R³⁴, and R³⁶ include, but are not limited to, the following.

^{∗}Hydrogen atom, hydroxyl group, thiol group, carboxyl group, nitro group, and cyano group;
^{∗}Halogen atoms such as fluorine atom, chlorine atom, bromine atom, and iodine atom;
^{∗}Alkyl groups such as methyl group, ethyl group, propyl group, isopropyl group, n-butyl group, isobutyl group, tert-butyl group, sec-butyl group, pentyl group, isopentyl group, neopentyl group, hexyl group, heptyl group, octyl group, decyl group, and nonyl group;
^{∗}Alkenyl groups such as ethenyl group, propenyl group, allyl group, butenyl group, pentenyl group, hexenyl group, heptenyl group, and octenyl group;
^{∗}Alkylyl groups such as propargyl group;
^{∗}Cycloalkyl groups such as cyclopropyl group, cyclobutyl group, cyclopentyl group, cyclohexyl group, cycloheptyl group, bicyclooctyl group, and spirooctyl group;
^{∗}Alkoxy groups such as methoxy group, ethoxy group, propoxy group, butoxy group, secbutoxy group, and tert-butoxy group;
^{∗}Aryl groups such as phenyl group, benzyl group, tolyl group, naphthyl group, and anthracenyl group;
^{∗}Aryloxy groups such as phenyloxy group, benzyloxy group, and naphthyloxy group;
^{∗}Acyl groups such as acetyl group, propionyl group, benzoyl group, p-methoxybenzoyl group, and cinnamoyl group;
^{∗}Unsubstituted amino group and substitution amino groups such as dimethyl amino group, benzyl amino group, and triphenylmethyl amino group;
^{∗}Heterocyclic groups such as furanyl group, thiophenyl group, pyranyl group, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, 2,5-dihydro-1,3-thiazolyl group, carbazolyl group; and
^{∗}Heterocyclic oxy groups such as furanyl oxy group, pyrrolyl oxy group, indolyl oxy group, and quinolyl oxy group.

Of the groups mentioned above, those having a carboxyl group may or may not have a protective group. When such a group with a carboxyl group has a protective group, the reaction selectivity with the carboxyl group on the right side of formula (3-1) is usually superior to that with the carboxyl groups present on the other substituents, although it may also depend on the reactivity of the compound (3-1) and the compound (3-2) used in the reaction.

R³³and R³⁶, independently of each other, represent a hydrogen atom, carboxyl group, or hydroxyl group, or, a monovalent hydrocarbon group or heterocyclic group that may have one or more substituents. The details of the substituents, if any, are described above. Examples of the number of such substituents include, for example, 5, 4, 3, 2, 1, or 0.

When each of R³³and/or R³⁶ is a monovalent hydrocarbon group or heterocyclic group that may have one or more substituents, then there may be an intervening linking group between the hydrocarbon group or heterocyclic group and the carbon atom to which the hydrocarbon group or heterocyclic group binds. Such linking group are not limited, but may be selected, independently of each other, from the structures indicated below (where, in the chemical formulae below, A represents a monovalent hydrocarbon group or a heterocyclic group, each of which may independently have one or more substituents. When two A's are present in the same group, they may be identical to each other or different from each other.).

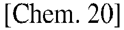

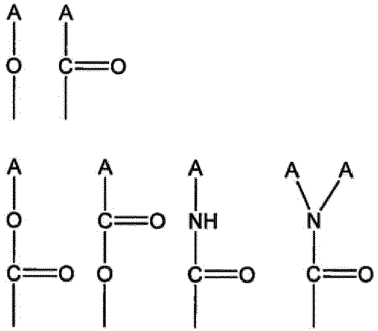

The upper limit of the number of carbon atoms hydrocarbon group (including its substituents, if any) may be, for example, 20 or less, 15 or less, 10 or less, 8 or less, or 6 or less. The lower limit thereof depends on the type of the hydrocarbon group, but may be 1 or more for alkyl groups, 2 or more for alkenyl and alkynyl groups, and 3 or more, such as 4 or more, or 5 or more, for cycloalkyl groups. Examples of the number of atoms include, for example, 1, 2, 3, 4, 5, 6, 7, 8, 9, 10, 11, 12, 13, 14, 15, 16, 17, 18, 19, or 20.

The upper limit of the total number of carbon atoms and hetero atoms of each heterocyclic group (including its substituents, if any) may be, for example, 20 or less, 15 or less, 10 or less, 8 or less, or 6 or less. The lower limit thereof depends on the type of the heterocyclic structure, but may typically be 3 or more, for example 4 or more, or 5 or more. Examples of the number of atoms include, for example, 3, 4, 5, 6, 7, 8, 9, 10, 11, 12, 13, 14, 15, 16, 17, 18, 19, or 20.

Among them, each of R³³and R³⁶ may preferably be selected, independently of each other, from hydrogen atom, hydroxyl group, or carboxyl group, or, that may have one or more substituents, alkyl group, alkenyl group, cycloalkyl group, alkoxy group, aryl group, aryloxy group, acyl group, heterocyclic group, and heterocyclic oxy group.

Examples of R³³and R³⁶ include, but are not limited to, the following.

^{∗}Hydrogen atom, hydroxyl group, and carboxyl group;
^{∗}Alkyl groups such as methyl group, ethyl group, propyl group, isopropyl group, n-butyl group, isobutyl group, tert-butyl group, sec-butyl group, pentyl group, isopentyl group, neopentyl group, hexyl group, heptyl group, octyl group, decyl group, and nonyl group;
^{∗}Alkenyl groups such as ethenyl group, propenyl group, allyl group, butenyl group, pentenyl group, hexenyl group, heptenyl group, and octenyl group;
^{∗}Alkylyl groups such as propargyl group;
^{∗}Cycloalkyl groups such as cyclopropyl group, cyclobutyl group, cyclopentyl group, cyclohexyl group, cycloheptyl group, bicyclooctyl group, and spirooctyl group;
^{∗}Aryl groups such as phenyl group, benzyl group, tolyl group, naphthyl group, anthracenyl group; and
^{∗}Heterocyclic groups such as furanyl group, thiophenyl group, pyranyl group, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, 2,5-dihydro-1,3-thiazolyl group, and carbazolyl group.

R³¹ and R³³ may be bound to each other to form, together with the carbon atom to which R³¹ binds and the nitrogen atom to which R³³ binds, a hetero ring that may have one or more substituents. Likewise, R³⁴ and R³⁶ may be bound to each other to form, together with the carbon atom to which R³⁴ binds and the nitrogen atom to which R³⁶ binds, a hetero ring that may have one or more substituents. The details of the substituents, if any, are described above. Examples of the number of such substituents include, for example, 5, 4, 3, 2, 1, or 0.

The upper limit of the total number of carbon atoms and hetero atoms of each heterocyclic group (including its substituents, if any) may be, for example, 20 or less, 15 or less, 10 or less, 8 or less, or 6 or less. The lower limit thereof depends on the type of the heterocyclic structure, but may typically be 3 or more, for example 4 or more, or 5 or more. Examples of the number of atoms include, for example, 3, 4, 5, 6, 7, 8, 9, 10, 11, 12, 13, 14, 15, 16, 17, 18, 19, or 20.

Examples of such hetero rings include, but are not limited to, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, and 2,5-dihydro-1,3-thiazolyl group.

A¹ to A⁴, independently of each other, represent a divalent aliphatic hydrocarbon group that may have one or more substituents having 1 to 3 carbon atoms. Examples include, but are not limited to, methylene group, ethylene group, propylene group, and isopropylene group, as well as any groups derived from these groups via substituition with one or more substituents. Examples of the number of such substituents include, for example, 3, 2, 1, or 0.

p1 to p4, independently of each other, represent an integer of 0 or 1.

m and n, independently of each other, represent an integer of equal to or greater than 1 corresponding to the number of the structure units parenthesized with [ ]. In other words, m represents the number of the amino acid residues parenthesized with [ ] in general formula (3-1). When m is 1, then the compound (3-1) is an amino acid, while when m is equal to or greater than 2, then the compound (3-1) is a peptide. Likewise, n represents the number of the amino acid residues parenthesized with [ ] in general formula (3-2). When n is 1, then the compound (3-2) is an amino acid, while n is equal to or greater than 2, then the compound (3-2) is a peptide. The upper limit of each of m and n is not particularly limited so long as the amidation step proceeds, but may preferably be, for example, 100 or less, 80 or less, 60 or less, 50 or less, 40 or less, 30 or less, 20 or less, 15 or less, 12 or less, or 10 or less. Examples of each of m and n, independently of each other, include 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, and 100.

Needless to say, when m is equal to or greater than 2, then each of R¹, R², R³, A¹, A², p1, and p2 in the structure parenthesized with [ ] may be either identical to each other or different from each other among the two or more amino acid residues. Likewise, when n is equal to or greater than 2, then each of R⁴, R⁵, R⁵, A³, A⁴, p3, and p4 in the structure parenthesized with [ ] may be either identical to each other or different from each other among the two or more amino acid residues. In other words, when each of the compound (3-1) and/or the compound (3-2) is a peptide, the two or more amino acid units constituting the peptide may be identical to each other or different from each other.

T¹ represents a hydrogen atom or a monovalent substituent. The type of the monovalent substituent is not particularly restricted, but may be selected from the examples of R³³ and R³⁶ mentioned above, as well as any protective groups for amino groups (hereinafter also referred to as PG¹). The protective group PG¹ for amino groups is not restricted as long as the amino group can be protected so that it does not react in the amidation process and can be deprotected and converted to an amino group after the reaction.

There are a wide variety of protective groups for amino groups, PG¹, known to the art. Examples thereof include monovalent hydrocarbon groups that may have one or more substituents and monovalent heterocyclic groups that may have one or more substituents. Preferred among these are monovalent hydrocarbon groups that may have one or more substituents. There may be any intervening linking group between the hydrocarbon group or heterocyclic group and the nitrogen atom of the amino group to be protected (the nitrogen atom in formula (3-1) to which PG¹ binds). Such a linking group is not particularly limited, but may be selected, independently of each other, from the linking groups listed below (where, in the chemical formulae below, A represents a monovalent hydrocarbon group or a heterocyclic group, each of which may independently have one or more substituents. When two A's are present in the same group, they may be identical to each other or different from each other.).

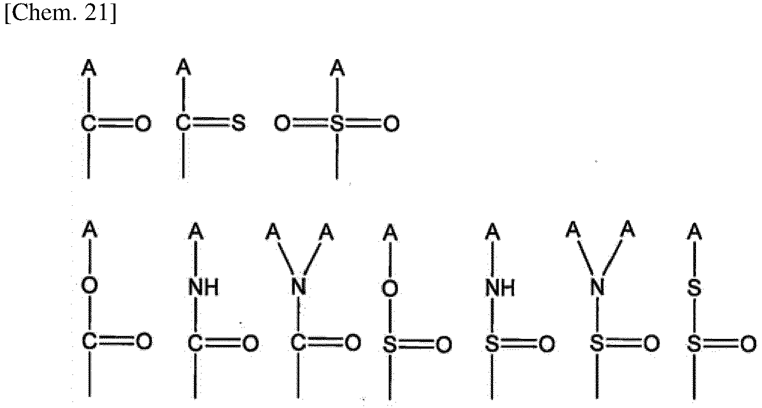

The number of carbon atoms in the protective group PG¹ may typically be 1 or more, or 3 or more, and typically 20 or less, or 15 or less.

Among them, the amino-protective group PG¹ may preferably be one or more selected from the group consisting of a monovalent hydrocarbon group, acyl group, hydrocarbon oxycarbonyl group, hydrocarbon sulfonyl group, and amide group that may have one or more substituents.

Specific examples of the amino-protective group PG¹ are listed below. Incidentally, an amino-protective group may be referred to either by the name of the functional group excluding the nitrogen atom of the amino group to which it binds or by the name of the group including the nitrogen atom to which it binds. The following list includes either or both of these names for each protective group.

Examples of unsubstituted or substituted hydrocarbon groups includes: alkyl groups such as methyl group, ethyl group, and propyl group; alkenyl groups such as ethenyl group, propenyl group, and allyl group; alkylyl groups such as propargyl group; cycloalkyl groups such as cyclopropyl group, cyclobutyl group, cyclopentyl group, and cyclohexyl group; aryl groups such as phenyl group, benzyl group, p-methoxybenzyl group, tolyl group, and triphenylmethyl group (Troc group); and substituted hydrocarbon groups such as cyanomethyl group. The number of carbon atoms may typically be 1 or more, or 3 or more, and typically 20 or less, or 15 or less.

Examples of unsubstituted or substituted acyl groups includes: benzoyl group (Bz), o-methoxybenzoyl group, 2,6-dimethoxy benzoyl group, p-methoxybenzoyl group (PMPCO), cinnamoyl group, and phthaloyl group (Phth).

Examples of unsubstituted or substituted hydrocarbon oxycarbonyl groups includes: tert-butoxycarbonyl group (Boc), benzyloxycarbonyl group (Cbz or Z), methoxycarbonyl group, ethoxycarbonyl group, 2-(trimethylsilyl)ethoxycarbonyl group, 2-phenyl ethoxycarbonyl group, 1-(1-adamanthyl)-1-methylethoxycarbonyl group, 1-(3,5-di-t-butylphenyl)-1-methylethoxycarbonyl group, vinyloxycarbonyl group, allyloxycarbonyl group (Alloc), N-hydroxypiperidinyloxycarbonyl group, p-methoxybenzyloxycarbonyl group, p-nitrobenzyloxycarbonyl group, 2-(1,3-dithianyl)methoxycarbonyl, m-nitrophenoxycarbonyl group, 3,5-dimethoxybenzyloxycarbonyl group, o-nitrobenzyloxycarbonyl group, 2,2,2-trichloroethoxycarbonyl group (Troc), and 9-fluorenylmethyloxycarbonyl group (Fmoc).

Examples of unsubstituted or substituted hydrocarbon sulfonyl groups includes: methanesulfonyl group (Ms), toluenesulfonyl group (Ts), and 2- or 4-nitro benzene sulfonyl (Ns) group.

Examples of unsubstituted or substituted amide groups includes: acetamide, o-(benzoyloxymethyl)benzamide, 2-[(t-butyl-diphenyl-siloxy)methyl]benzamide, 2-toluenesulfonamide, 4-toluenesulfonamide, 2-nitro benzene sulfonamide, 4-nitro benzene sulfonamide, tert-butylsulfinyl amide, 4-toluenesulfonamide, 2-(trimethylsilyl)ethanesulfonamide, and benzyl sulfonamide.

In terms of deprotection methods, the protective group PG¹ may be deprotected by, e.g., at least one of the following methods: deprotection by hydrogenation, deprotection by weak acid, deprotection by fluorine ion, deprotection by one-electron oxidizing agent, deprotection by hydrazine, and deprotection by oxygen.

Preferred examples of the amino protective group PG¹ include mesyl group (Ms), tert-butoxycarbonyl group (Boc), benzyl group (Bn or Bzl), benzyloxycarbonyl group (Cbz), benzoyl group (Bz), p-methoxybenzyl group (PMB), 2,2,2-trichloroethoxycarbonyl group (Troc), allyloxycarbonyl group (Alloc), 2,4-dinitrophenyl group (2,4-DNP), phthaloyl group (Phth), p-methoxybenzoyl group (PMPCO), cinnamoyl group, toluenesulfonyl group (Ts), 2- or 4-nitrobenzenesulfonyl group (Ns), cyanomethyl group, and 9-fluorenylmethyloxycarbonyl group (Fmoc). These protective groups are preferred because, as mentioned above, they can easily protect the amino group and can be removed under relatively mild conditions.

More preferable examples of the amino protective group PG¹ include mesyl group (Ms), tert-butoxycarbonyl group (Boc), benzyloxycarbonyl group (Cbz), benzyl group (Bn), p-methoxybenzyl group (PMB), 2,2,2-trichloroethoxycarbonyl group (Troc), allyloxycarbonyl group (Alloc), p-methoxybenzoyl group (PMPCO), benzoyl group (Bz), cyanomethyl group, cinnamoyl group, 2- or 4-nitrobenzenesulfonyl group (Ns), toluenesulfonyl group (Ts), phthaloyl group (Phth), 2,4-dinitrophenyl group (2,4-DNP), and 9-fluorenylmethyloxycarbonyl group (Fmoc).

Even more preferable examples of the amino protective group PG¹ include mesyl group (Ms), tert-butoxycarbonyl group (Boc), benzyloxycarbonyl group (Cbz), benzyl group (Bn), p-methoxybenzyl group (PMB), 2,2,2-trichloroethoxycarbonyl group (Troc), allyloxycarbonyl group (Alloc), p-methoxybenzoyl group (PMPCO), benzoyl group (Bz), cyanomethyl group, and cinnamoyl group.

T² represents a hydrogen atom or a monovalent substituent. The type of the monovalent substituent is not particularly limited, but may be selected from the examples of R³¹, R³², R³⁴, and R³⁶ mentioned above, as well as protective groups for carboxyl group (hereinafter also referred to as PG²). The carboxyl protective group PG² is not limited as long as the carboxyl group can be protected so that it does not react in the amidation process, and then can be deprotected and converted to a carboxyl group after the reaction.

Examples of the carboxyl protective group PG² include monovalent hydrocarbon groups or heterocyclic groups that may have one or more substituents. The details of the substituents, if any, are described above. Examples of the number of such substituents include, for example, 5, 4, 3, 2, 1, or 0.

The upper limit of the number of carbon atoms of each hydrocarbon group (including its substituents, if any) may be for example 20 or less, 15 or less, 10 or less, 8 or less, or 6 or less. The lower limit thereof depends on the type of the hydrocarbon group, but may be 1 or more for alkyl groups, 2 or more for alkenyl and alkynyl groups, and 3 or more, such as 4 or more, or 5 or more, for cycloalkyl groups. Examples of the number of atoms include, for example, 1, 2, 3, 4, 5, 6, 7, 8, 9, 10, 11, 12, 13, 14, 15, 16, 17, 18, 19, or 20.

The upper limit of the total number of carbon atoms and hetero atoms of each heterocyclic group (including its substituents, if any) may be, for example, 20 or less, 15 or less, 10 or less, 8 or less, or 6 or less. The lower limit thereof depends on the type of the heterocyclic structure, but may typically be 3 or more, for example 4 or more, or 5 or more. Examples of the number of atoms include, for example, 3, 4, 5, 6, 7, 8, 9, 10, 11, 12, 13, 14, 15, 16, 17, 18, 19, or 20.

Examples of the carboxyl protective group PG² include, but are not limited to, the following.

^{∗}Alkyl groups such as methyl group, ethyl group, propyl group, isopropyl group, n-butyl group, isobutyl group, tert-butyl group, sec-butyl group, pentyl group, isopentyl group, neopentyl group, hexyl group, heptyl group, octyl group, decyl group, and nonyl group;
^{∗}Alkenyl groups such as ethenyl group, propenyl group, allyl group, butenyl group, pentenyl group, hexenyl group, heptenyl group, and octenyl group;
^{∗}Alkylyl groups such as propargyl group;
^{∗}Cycloalkyl groups such as cyclopropyl group, cyclobutyl group, cyclopentyl group, cyclohexyl group, cycloheptyl group, bicyclooctyl group, and spirooctyl group;
^{∗}Aryl groups such as phenyl group, benzyl group, tolyl group, naphthyl group, anthracenyl group;
^{∗}Heterocyclic groups such as furanyl group, thiophenyl group, pyranyl group, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, 2,5-dihydro-1,3-thiazolyl group, and carbazolyl group; and
^{∗}Silicon-based protective groups such as trimethylsilyl (TMS) group, triethylsilyl (TES) group, triisopropylsilyl (TIPS) group, tri(tert-butyl)silyl (TBS) group, tertbutyldiphenylsilyl (TBDPS) group and tris(trialkylsilyl)silyl group.

With regard to the compound (3-2), the amino group on the left side of general formula (3-2) may form a salt with an acid. In this case, examples of the acid include, but are not limited to' aliphatic carboxylic acids with 1 to 5 carbon atoms, such as acetic acid and propionic acid; and trifluoroacetic acid, hydrochloric acid, hydrobromic acid, sulfuric acid, nitric acid, phosphoric acid, boric acid, and sulfonic acid.

Embodiment (3) is deemed a specific embodiment of Embodiment (1) in which the compound (1-1) and the compound (1-2) are both amino acids or peptides and the compound (1-3) is a peptide. Likewise, Embodiment (2) has a specific embodiment in which the compound (2-1) is an amino acid or peptide and the compound (2-3) is a cyclic peptide. The details of such a specific embodiment are clear to those skilled in the art, taking into account the details of Embodiments (2) and (3) explained above.

In all of these embodiments, some or all of the reactant compounds may be linked or immobilized to a reactant, resin, or other carrier at any of their substituents. In this case, the type of the reactant, resin, or other carrier is not limited. Any known reactant, resin, or other carrier can be used as long as it does not substantially interfere with the amide reaction in the production method according to the present invention and does not depart from the purpose of the present invention. There are no restrictions the manner of linking and immobilizing the reactant compound to the reactant, resin, or other carrier, although it is preferable to form a covalent bond between any substituent of the reactant compound and any substituent present on the reactant, resin, or other carrier. There are also no restrictions on the type of each substituent and the method of forming a covalent bond. Any known substituent and covalent bond formation method can be used as long as it does not substantially interfere with the amide bond reaction in the production method according to the present invention and does not depart from the purpose of the present invention. When the reactant compounds are amino acids or peptides, such as in the case of Embodiment (3), the reactant compound may be linked and immobilized to a reactant, resin, or other carrier by covalent bonding using a carboxyl or amino group of the reactant compound (other than the carboxyl ester or amino group being the target of the amide reaction). Such a mode can be regarded as a variation of the embodiment in which the carboxyl or amino group of the reactant compound (other than the carboxyl ester or amino group being the target of the amide reaction) is protected by introducing a protective group.

### ^{∗}Amount(s) of the reactant compound(s) used:

The amounts of the reactant compounds used in the production method according to the present invention depend on the embodiment of the production method according to the present invention, but are generally as follows.

In the case of Embodiment (1), the amount ratio between the compound (1-1) and the compound (1-2) is not particularly limited, but may be determined as follows. The molar ratio of the compound (1-2) to 1 mol of the compound (1-1) may be typically 0.1 mol or more, for example 0.2 mol or more, 0.3 mol or more, or 0.5 mol or more, and may be typically 20 mol or less, for example 10 mol or less, 8 mol or less, 6 mol or less, 5 mol or less, or 3 mol or less. Depending on the type of Brønsted acid used as a catalyst, the Brønsted acid itself may cause an amidation reaction. Therefore, it is preferable to use the compound (1-2) with an amino group in a larger amount than that of the compound (1-1) with a carboxyl ester group in order to improve reaction efficiency. Specifically, the molar ratio of the compound (1-2) to 1 mol of the compound (1-1) may be 1 or more, particularly 1.5 or more. Needless to say, it is necessary to use at least 1 mol of each of the reactant compounds (1-1) and (1-2) for 1 mol of the target compound (1-3) to be produced.

In the case of Embodiment (3), the amount ratio between the compound (3-1) and the compound (3-2) is the same as in Embodiment (1). Specifically, although the ratio in Embodiment (3) is not particularly limited to, the molar ratio of the compound (3-2) to 1 mol of the compound (3-1) may typically be 0.1 mol or more, for example 0.2 mol or more, 0.3 mol or more, or 0.5 mol or more, and also may be typically 20 mol or less, for example 10 mol or less, 8 mol or less, 6 mol or less, 5 mol or less, or 2 mol or less. Depending on the type of Brønsted acid used as a catalyst, the Brønsted acid itself may cause an amidation reaction. Therefore, it is preferable to use the compound (3-2) with an amino group in a larger amount than that of the compound (3-1) with a carboxyl ester group in order to improve reaction efficiency. Specifically, the molar ratio of the compound (3-2) to 1 mol of the compound (3-1) may be 1 or more, particularly 1.5 or more. Needless to say, it is necessary to use at least 1 mol of each of the reactant compounds (3-1) and (3-2) for 1 mol of the target compound (3-3) to be produced.

### ^{∗}Amount of the catalyst used:

Although not particularly limited, when each reactant compound is used in 100 mol%, the amount of the Brønsted acid used as the catalyst of this invention may be, e.g., typically 1 mol% or more, particularly 3 mol% or more, or 5 mol% or more, or 10 mol% or more, and typically 500 mol% or less, particularly 300 mol% or less, or 200 mol% or less, or 100 mol% or less. Especially when no other solvent is used and the Brønsted acid is also used as a reaction medium, a sufficient amount of the Brønsted acid may be used to sufficiently dissolve or disperse each reactant compound.

### ^{∗}Other ingredients:

In the production method according to the present invention, the reaction system may contain one or more other ingredients, in addition to the reactant compound(s) (i.e., the compounds (1-1) and (1-2) in Embodiment (1), the compound (2-1) in Embodiment (2), and the compounds (3-1) and (3-2) in Embodiment (3)) and the Brønsted acid.

For example, the amidation reaction may be carried out in the presence of a base from the viewpoint of increasing reaction efficiency. Bases are not restricted, but may include, for example, triethylamine (Et3N), diisopropylamine (i- Pr₂NH), diisopropylethylamine (i-Pr2EtN) and other amines having 1 to 3 linear- or branched-chain alkyl groups each having 1 to 10 carbons. The amount of the base used is not particularly restricted, but for example, when each reactant compound is used in an amount of 100 mol%, the base may be used in an amount of from 20 to 120 mol%, or from 50 to 100 mol%. However, since the Brønsted acid as the reaction medium for dissolving or dispersing each reactant compound in the present invention, it is rather preferable not to use any solvents other than the Brønsted acid from the viewpoint of reaction efficiency.

The reaction mixture may also contain other optional components such as conventional catalysts, silane compounds, phosphorus compounds, etc., that can be used in the amidation reaction. For the details of such optional components, it is possible to refer, for example, to the descriptions of the earlier patent applications filed by the present inventors, which will be mentioned later.

### ^{∗}Reaction procedure:

Amidation by the production method according to the present invention can be carried out by contacting the reactant compound(s) with the Brønsted acid, and optionally with the other ingredients if used. The order of contacting these components is not limited, and all may be mixed simultaneously or sequentially in any order. Specific examples include, but are not limited to, the following procedures.

For example, if there are no other carboxyl groups, amino groups, and/or other reactive groups than the carboxyl ester and amino groups being the target of the desired amidation reaction, or if such other carboxyl groups, amino groups, or other reactive groups are protected with protective groups, the order of contact is not particularly limited. However, from the standpoint of reaction efficiency, it is preferred to mix the reactant compounds and the Brønsted acid and/or the other components. When each of other carboxyl groups and/or amino groups than the target carboxyl and amino groups is protected with a protective group, it would be necessary to carry out a protection step to introduce the protective group before the amidation reaction, and also a deprotection step to remove the protective group after the amidation reaction.

From the viewpoint of increasing reaction efficiency, amidation may be carried out in an organic solvent. The type of the organic solvent is not particularly limited, examples thereof including: aromatic hydrocarbons such as toluene and xylene; pentane; ethers such as petroleum ether, 1-methyltetrahydrofuran (1-MeTHF), diisopropylether (i-Pr₂O), diethylether (Et₂O), cyclopentylmethylether (CPME): esters such as ethyl acetate (AcOEt); and organic acids such as acetic acid. These organic solvents may be used either singly or in combination of any two or more.

The concentration of each reactant compound in the reaction system is not restricted, but from the viewpoint of increasing reaction efficiency, it may be between 2 volume% and 70 volume%.

### ^{∗}Reaction conditions:

The conditions for the amidation reaction in the production method according to the present invention are not limited as long as the reaction proceeds, although examples of the conditions for each reaction step are described below.

When the reactant compound(s) is mixed with the Brønsted acid simultaneously, along with optionally-used other ingredients, the reaction condition are not limited as long as the reaction proceeds, examples thereof being as follows.

The reaction temperature is not limited as long as the reaction proceeds, although it may typically be 0°C or more, particularly 10°C or more, especially 20°C or more, and also may typically be 120°C or less, particularly 100°C or less, especially 80°C or less. In this regard, the production method according to the present invention is advantageous because the amidation reaction sufficiently proceeds even under a mild condition, e.g., at a temperature of 60°C or less.

The reaction pressure is not limited as long as the reaction proceeds, and the reaction may be carried out under reduced, normal, or pressurized pressure, but may typically be carried out under normal pressure.

The reaction atmosphere is also not limited as long as the reaction proceeds, but the reaction may be carried out under an atmosphere of inert gas such as argon or nitrogen.

The reaction time is also not limited as long as the reaction proceeds. However, in order for the reaction to proceed sufficiently and efficiently, the reaction time may be 10 minutes or more, particularly 20 minutes or more, or 30 minutes or more, and may be 120 hours or less, particularly 100 hours or less, or 80 hours or less.

In all of these embodiments, the production method according to the present invention (or, if the method includes two or more steps, each of the steps) may be carried out in a sequential manner (batch) or in a continuous manner (flow). Details of specific procedures for implementing a sequential method (batch method) and a continuous method (flow method) are well known in the art.

### ^{∗}Embodiment using reactant compounds having aromatic or heterocyclic ester group:

A preferred embodiment of the production method of the present invention relates to an embodiment in which the ester-forming group R¹⁰, R²⁰, or R³⁰ of the reactant compound represented by general formula (1-1), (2-1), or (3-1), which group is the subject of the amidation reaction, is a monovalent aromatic hydrocarbon group or heterocyclic group which may have one or more substituents (i.e., an embodiment in which R¹⁰, R²⁰, or R³⁰ forms an aromatic or heterocyclic ester). Reaction in the presence of the Brønsted acid using such reactant compounds represented by general formula (1-1), (2-1), or (3-1) in which the ester-forming group R¹⁰, R²⁰, or R³⁰ forms an aromatic or heterocyclic ester may enable amidation at lower temperatures and/or in a shorter time.

According to this embodiment, the aromatic hydrocarbon group or heterocyclic group used as R¹⁰, R²⁰, or R³⁰ may be arbitrary chosen. The type of aromatic or heterocyclic ring and the presence/absence, type, and number of substituents are also not restricted, examples thereof including as follows.

Examples of the aromatic hydrocarbon groups may include, although not limited to, the various aryl groups exemplified above, among which phenyl group may be preferred.

Examples of the heterocyclic groups may include, although not limited to, the various heterocyclic groups exemplified above, among which succinimide groups may be preferred.

If the aromatic hydrocarbon or heterocyclic group has one or more substituents, examples of the optional substituents may include, although not limited to, the various substituents exemplified above. Preferred among these are electron-withdrawing groups, among which halogen groups, nitro group, and methyl group are particularly preferred.

It is not clear as to why the use of the reactant compound of general formula (1-1), (2-1), or (3-1) in which R¹⁰, R²⁰, or R³⁰ forms an aromatic or heterocyclic ester in this embodiment enables a significant improvement in the efficiency of the amidation reaction. However, it is assumed to be because the synergistic effect of using a highly reactive amino acid leaving group in combination with the Brønsted acid may radically increase reaction efficiency.

The production method according to this embodiment may include, before the amidation reaction process in the presence of Brønsted acid as the catalyst of the present invention, the step of esterifying a carboxylic acid compound corresponding to the reactant compound of general formula (1-1), (2-1), or (3-1) (i.e., a compound where R¹⁰, R²⁰, or R³⁰ is a hydrogen atom) with an aromatic alcohol compound or heterocyclic alcohol compound to prepare the reactant compound being the subject to the amidation reaction.

In this embodiment, the aromatic or heterocyclic alcohol compound used for esterification of the carboxyl group is not limited, and may be selected according to the desired structure of the R¹⁰, R²⁰, or R³⁰ group.

Specific examples of aromatic or heterocyclic alcohol compounds used in this embodiment include: phenol compounds such as phenol, nitrophenol (o-, m-, or p-nitrophenol), bromophenol (o-, m-, or p-bromophenol), cresol (o-, m-, or p-methylphenol); and nitrogen-containing heterocyclic compounds such as N-hydroxysuccinimide. Preferred among these include phenol compounds and hydroxysuccinimide compounds, especially preferred specific examples including phenol, p-nitrophenol, p-bromophenol, m-cresol, and N-hydroxysuccinimide.

The method of esterifying the carboxyl group is not limited, and various conventionally known methods can be selected and used as appropriate. Examples of known methods of carboxyl group esterification include the Steklich esterification method. Steklich esterification method uses N,N'-dicyclohexylcarbodiimide (DCC) as a condensation reagent and N,N-dimethyl-4-aminopyridine (DMAP) as a catalyst. Examples of other condensation reagents that can be used include 1-ethyl-3-(3-dimethyl amino propyl) carbodiimide hydrochloride (water-soluble carbodiimide hydrochloride, WSCDHCl). In order to improve reactivity and control side reactions, various additives may also be used, such as 1-hydroxybenzotriazole (HoBt), N-hydroxysuccinimide (HOSu), 1-hydroxybenzotriazole (HoBt), 4-dimethylaminopyridine (DMAP), etc.

The use of other additives, the presence or absence and type of solvents, and various conditions such as temperature, atmosphere, pressure, and time during the reaction are also not limited and can be determined by a person skilled in the art in view of the technical common knowledge.

The reactant compound whose carboxyl group has been esterified by the above procedure, is then subjected to an amidation reaction in the presence of the Brønsted acid as the catalyst of the present invention. The details of such amidation reaction are as described previously. By esterifying the carboxyl group as described above before the amidation reaction in the presence of Brønsted acid, the reaction temperature can be significantly reduced (e.g., to 50°C or less, particularly 40°C or less, or even particularly 35°C or less) and/or the reaction time can be significantly reduced (e.g., to within 5 hours, among others, to 4 hours, or even 3 hours), whereby the reaction may be much more efficient.

### ^{∗}Post-processing, etc. (e.g., purification and recovery):

The production method according to the present invention may include the step of carrying out various post-processing onto the amido compound produced via the amidation reaction.

For example, the amide compound formed may be isolated and purified according to conventional methods such as column chromatography, recrystallization, etc.

When T¹ is the protective group PG¹ and/or T² is the protective group PG², the amino group protected by PG¹ and/or the carboxyl group protected by PG² of the amide compound produced may be deprotected, optionally after post-processing steps such as isolation and purification.

The method of deprotecting the amino group protected by PG¹ is not particularly restricted, and various methods can be used depending on the type of protecting group PG¹. Examples include deprotection by hydrogenation, deprotection by weak acids, deprotection by fluorine ions, deprotection by one-electron oxidants, deprotection by hydrazine, and deprotection by oxygen. The deprotection by hydrogenation may be carried out by, e.g.; (a) a method of causing deprotection in the presence of hydrogen gas using a metal catalyst such as palladium, palladium-carbon, palladium hydroxide, palladium-carbon hydroxide, etc., as a reduction catalyst; and (b) a method of causing deprotection in the presence of a metal catalyst such as palladium, palladium-carbon, palladium hydroxide, palladium-carbon hydroxide, etc., using a hydrotreating reductant such as sodium borohydride, lithium aluminum hydride, lithium borohydride, diborane, etc.

The method of deprotecting the carboxyl group protected by PG² is not particularly restricted, and various methods can be used depending on the type of protecting group PG². Examples include deprotection by hydrogenation, deprotection by bases, and deprotection by weak acids. In the case of deprotection with a base, a strong base such as lithium hydroxide, sodium hydroxide, potassium hydroxide, etc. can be used.

After conducting the amidation reaction by the production method according to the present invention, the resulting amide compound (if necessary, after deprotection) can be subjected to the production method according to the present invention again as a new reactant compound and linked to another reactant compound by an amide bond.

For example, in the case of Embodiment (3), a peptide obtained via an amidation reaction through the production method according to the present invention can be subjected to the production method according to the present invention to thereby cause an amidation reaction between the terminal amino or carboxyl group of the peptide and the terminal carboxyl or amino group of another amino acid or peptide to produce a novel peptide. Thus, the production method according to the present invention can be repeated in sequence, whereby a peptide having any amino acid sequence can be synthesized in principle.

Alternatively, after an amidation reaction is carried out via the production method according to the present invention, the resulting peptide may be bound to another amino acid using any other amidation methods. An example of such other amidation methods is the method described in WO 2018/199147 A (PTL 3), which was also developed by the present inventors. The method described in WO 2018/199147 A is a method for forming an amide bond between the carboxyl group of a first amino acid or peptide and the amino group of a second amino acid in the presence of a metal catalyst such as a specific tantalum or niobium compound. Specifically, in the case of Embodiment (3), the carboxyl protective group PG² of the compound (3-2) may be selected from the protective groups that can react in the presence of a metal catalyst such as a certain tantalum or niobium compound used in the method described in WO 2018/199147 A. This compound (3-2) may be used in the production method according to the present invention to thereby produce the compound (3-3), which may be subjected to the method described in WO 2018/199147 A, thereby linking the compound (3-3) with another amino acid via amidation reaction.

The present inventors have filed the prior patent applications concerning various amidation reactions as indicated below. It is possible to carry out the amidation reaction by the production method of the present invention in combination with the amidation reactions described in these prior patent applications as appropriate, and/or to modify the amidation reaction by considering the conditions of the amidation reaction described in these prior patent applications as appropriate. The contents of these prior patent applications are incorporated herein by reference in their entirety.
(1) International Publication No. WO2017/204144 A (filed on May 22, 2017)
(2) International Publication No. WO2018/199146 A (filed on April 25, 2018)
(3) International Publication No. WO2018/199147 A (filed on April 25, 2018)
(4) International Publication No. WO2019/208731 A (filed on April 25, 2019)
(5) International Application No. PCT/JP2020/040951 (filed on October 30, 2020)
(6) International Application No. PCT/JP2020/040960 (filed on October 30, 2020)

### EXAMPLES

The present invention will be described in more detail below with reference to examples. However, the present invention should in no way be bound by the following examples, and can be implemented in any form within the scope that does not depart from the purpose of the invention.

Amide compounds were produced by the production method according to the present invention, in a manner described in each of the following examples.

In the following examples, unless otherwise stated, diastereomeric (dr) and enantiomeric (er) ratios are calculated by ¹H- NMR analysis (measuring instrument: JEOL 4 00SS, measurement conditions: 400 MH z, solvent: CDCl₃ or High performance liquid chromatography (HPLC) analysis using a DAICEL chiral column (measuring instrument: JEOL 400SS, measurement conditions: 400 MHz, solvent: CDCl³) or HPLC analysis (measuring instrument: Shimadzu SPD-M20A) using DAICEL chiral column. The dr and er values in the text are rounded off to one decimal place.

In the following examples, the reactant compound with a carboxylic ester group subject to the amidation reaction may be referred to as "Reactant Compound 1" and the reactant compound with an amino group subject to the amidation reaction as "Reactant Compound 2."

### [Example Group A: Amidation reactions using various Brønsted acids]

### ^{∗}General synthesis procedure A:

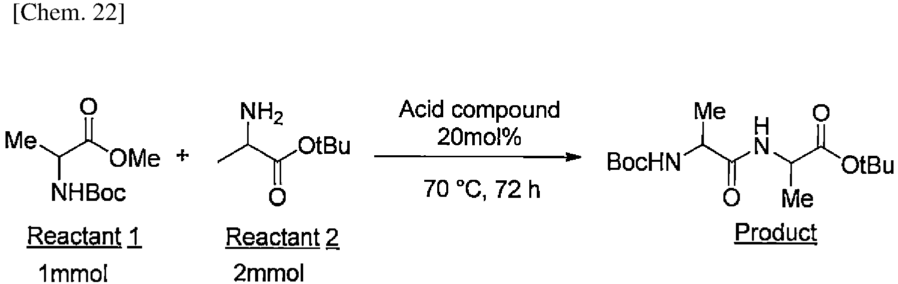

Under argon atmosphere, in a glove box, a heat-dried 5.0-mL screw-cap vial containing a stirrer (samarium-cobalt) was charged with Boc-L-Ala-OMe as Reactant Compound 1 and L-Ala-Ot-Bu as Reactant Compound 2 at ratios of Reactant Compound 1 = 1.0 mmol and Reactant Compound 2 = 2.0 mmol. 20 mol% of one of various acidic compounds was then added to the vial, and the reaction mixture was stirred vigorously at 70°C for 72 hours in a preheated oil bath under nitrogen atmosphere, then the reaction was cooled to ambient temperature. The reaction mixture was diluted with CHCl₃ (3.0 mL) and pipetted into a transferred to a silica gel column, and the used vial and pipette were washed with CHCl₃ (12.0 mL). The used vial and pipette were washed with CHCl₃ (12.0 mL). The reaction mixture was purified by flash column chromatography (using 0 to 60% AcOEt in hexane as mobile phase) to thereby afford the target compound Boc-L-Ala-L-Ala-Ot-Bu as a colorless liquid.

### ^{∗}Example A1: Reaction using acetic acid

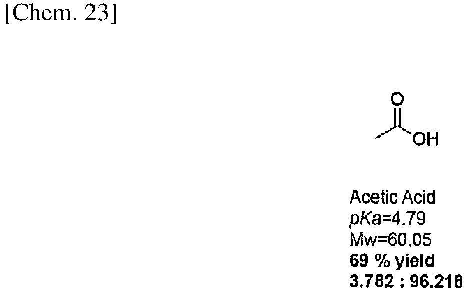

The reaction was carried out in accordance with General synthesis procedure A using acetic acid (pKa=4.79, Mw=60.05) as the acidic compound to thereby afford the target compound. Yield: 69%, dr = 96.2:3.8.

### ^{∗}Example A2: Reaction using propionic acid

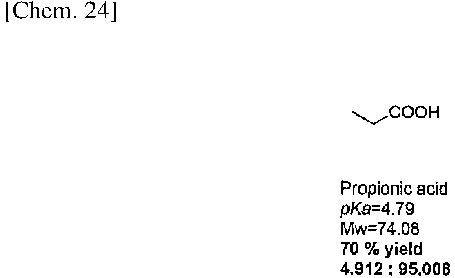

The reaction was carried out in accordance with General synthesis procedure A using propionic acid (pKa=4.79, Mw=74.08) as the acidic compound to thereby afford the target compound. Yield: 70%, dr = 95.0:4.9.

### ^{∗}Example A3: Reaction using butanoic acid

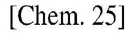

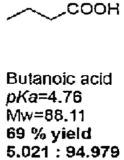

The reaction was carried out in accordance with General synthesis procedure A using butanoic acid (pKa=4.76, Mw=88.11) as the acidic compound to thereby afford the target compound. Yield: 69%, dr = 95.0:5.0.

### ^{∗}Example A4: Reaction using valeric acid

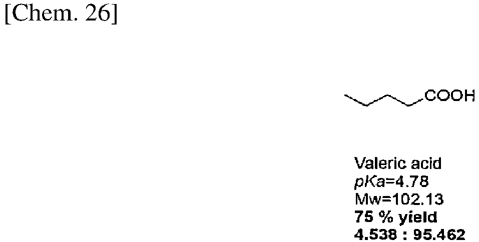

The reaction was carried out in accordance with General synthesis procedure A using valeric acid (pKa=4.78, Mw=102.13) as the acidic compound to thereby afford the target compound. Yield: 75%, dr = 95.5:4.5.

### ^{∗}Example A5: Reaction using caproic acid

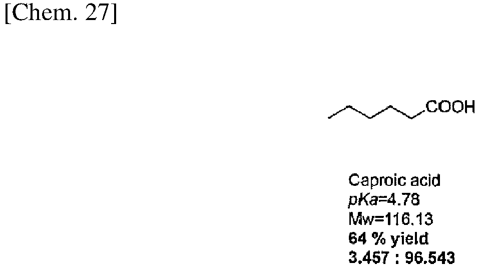

The reaction was carried out in accordance with General synthesis procedure A using caproic acid (pKa=4.78, Mw=116.13) as the acidic compound to thereby afford the target compound. Yield: 64%, dr = 96.5:3.5.

### ^{∗}Example A6: Reaction using thioacetic acid

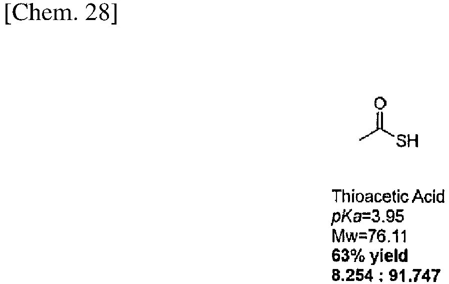

The reaction was carried out in accordance with General synthesis procedure A using thioacetic acid (pKa=3.95, Mw=76.11) as the acidic compound to thereby afford the target compound. Yield: 63%, dr = 91.7:8.3.

### ^{∗}Example A7: Reaction using formic acid

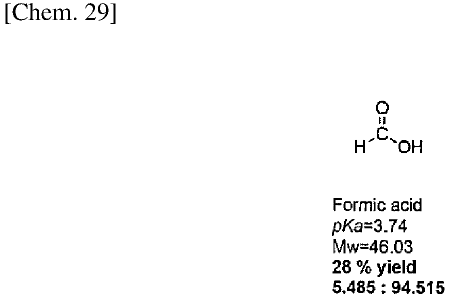

The reaction was carried out in accordance with General synthesis procedure A using formic acid (pKa=3.74, Mw=46.03) as the acidic compound to thereby afford the target compound. Yield: 28%, dr = 94.5:5.5.

### ^{∗}Example A8: Reaction using trichlorolic acid

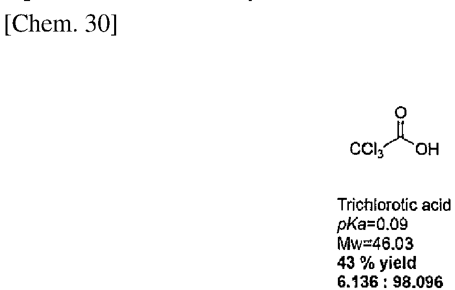

The reaction was carried out in accordance with General synthesis procedure A using trichlorolic acid (pKa=0.009, Mw=46.03) as the acidic compound to thereby afford the target compound. Yield: 43%, dr = 98.1:6.1.

### ^{∗}Example A9: Reaction using trifluoroacetic acid

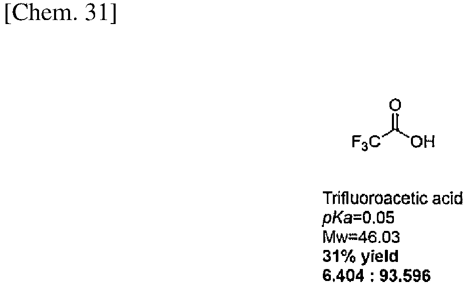

The reaction was carried out in accordance with General synthesis procedure A using trifluoroacetic acid (pKa=0.05, Mw=46.03) as the acidic compound to thereby afford the target compound. Yield: 31%, dr = 93.6:6.4.

### ^{∗}Example A10: Reaction using ammonium acetate

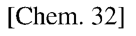

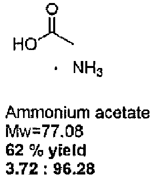

The reaction was carried out in accordance with General synthesis procedure A using ammonium acetate (pKa=9.9, Mw=77.08) as the acidic compound to thereby afford the target compound. Yield: 62%, dr = 96.3:3.7.

### ^{∗}Example A11: Reaction using isobutyric acid

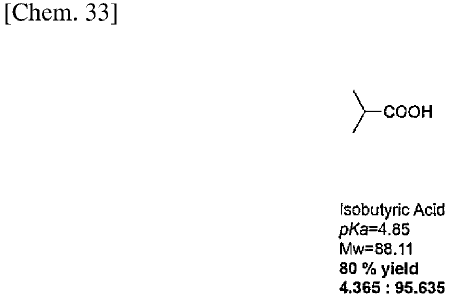

The reaction was carried out in accordance with General synthesis procedure A using isobutyric acid (pKa=4.85, Mw=88.11) as the acidic compound to thereby afford the target compound. Yield: 80%, dr = 95.6:4.4.

### ^{∗}Example A12: Reaction using pivalic acid

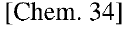

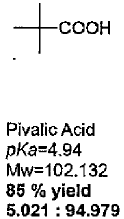

The reaction was carried out in accordance with General synthesis procedure A using pivalic acid (pKa=4.94, Mw=102.132) as the acidic compound to thereby afford the target compound. Yield: 85%, dr = 95.0:5.0.

### ^{∗}Example A13: Reaction using diisopropylacetic acid

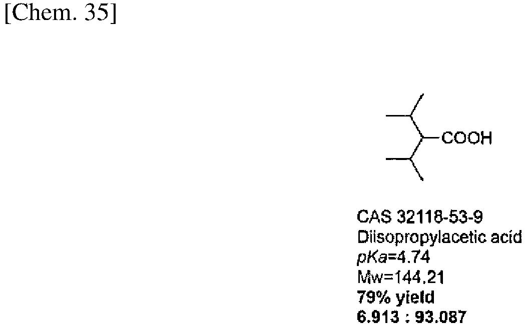

The reaction was carried out in accordance with General synthesis procedure A using diisopropylacetic acid (pKa=4.74, Mw=144.21) as the acidic compound to thereby afford the target compound. Yield: 79%, dr = 93.1:6.9.

### ^{∗}Example A14: Reaction using 2-tert-butyl-3,3-dimethylbutanoic acid

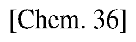

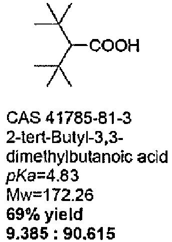

The reaction was carried out in accordance with General synthesis procedure A using 2-tert-butyl-3,3-dimethylbutanoic acid (pKa=4.83, Mw=172.26) as the acidic compound to thereby afford the target compound. Yield: 69%, dr = 90.6:9.4.

### ^{∗}Example A15: Reaction using oxalic acid

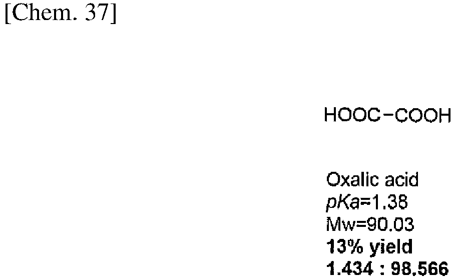

The reaction was carried out in accordance with General synthesis procedure A using oxalic acid (pKa=1.38, Mw=90.03) as the acidic compound to thereby afford the target compound. Yield: 13%, dr = 98.6:1.4.

### ^{∗}Example A16: Reaction using malonic acid

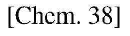

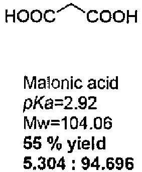

The reaction was carried out in accordance with General synthesis procedure A using malonic acid (pKa=2.92, Mw=104.06) as the acidic compound to thereby afford the target compound. Yield: 55%, dr = 94.7:5.3.

### ^{∗}Example A17: Reaction using succinic acid

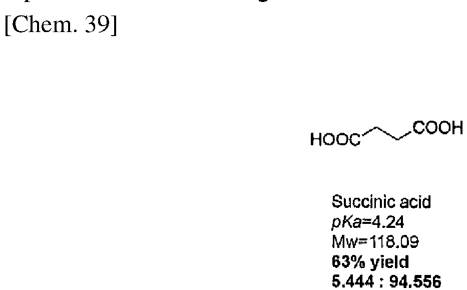

The reaction was carried out in accordance with General synthesis procedure A using succinic acid (pKa=4.24, Mw=118.09) as the acidic compound to thereby afford the target compound. Yield: 63%, dr = 94.6:5.4.

### ^{∗}Example A18: Reaction using L-malic acid

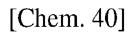

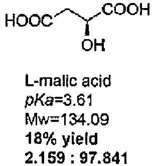

The reaction was carried out in accordance with General synthesis procedure A using L-malic acid (pKa=3.61, Mw=134.09) as the acidic compound to thereby afford the target compound. Yield: 18%, dr = 97.8:2.2.

### ^{∗}Example A19: Reaction using adipic acid

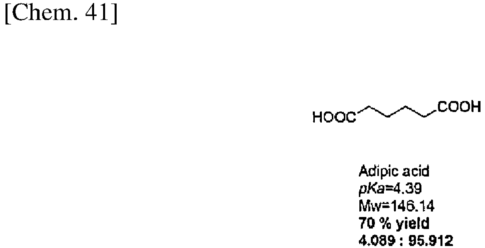

The reaction was carried out in accordance with General synthesis procedure A using adipic acid (pKa=4.39, Mw=146.14) as the acidic compound to thereby afford the target compound. Yield: 70%, dr = 95.9:4.1.

### ^{∗}Example A20: Reaction using fumaric acid

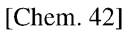

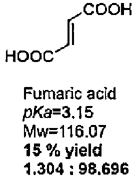

The reaction was carried out in accordance with General synthesis procedure A using fumaric acid (pKa=3.15, Mw=116.07) as the acidic compound to thereby afford the target compound. Yield: 15%, dr = 98.7:1.3.

### ^{∗}Example A21: Reaction using lactic acid

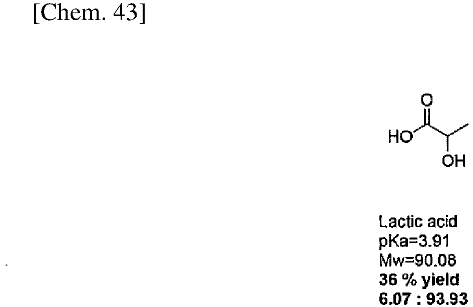

The reaction was carried out in accordance with General synthesis procedure A using lactic acid (pKa=3.91, Mw=90.08) as the acidic compound to thereby afford the target compound. Yield: 36%, dr = 93.9:6.1.

### ^{∗}Example A21(a): Reaction using L-lactic acid

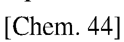

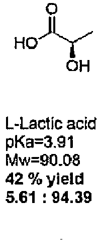

The reaction was carried out in accordance with General synthesis procedure A using L-lactic acid (pKa=3.91, Mw=90.08) as the acidic compound to thereby afford the target compound. Yield: 42%, dr = 94.4:5.6.

### ^{∗}Example A21(b): Reaction using D-lactic acid

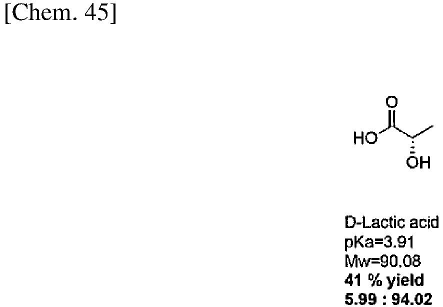

The reaction was carried out in accordance with General synthesis procedure A using D-lactic acid (pKa=3.91, Mw=90.08) as the acidic compound to thereby afford the target compound. Yield: 41%, dr = 94.0:6.0.

### ^{∗}Example A22: Reaction using L-tartaric acid

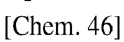

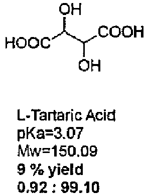

The reaction was carried out in accordance with General synthesis procedure A using L-tartaric acid (pKa=3.07, Mw=150.09) as the acidic compound to thereby afford the target compound. Yield: 9%, dr = 99.1:0.9.

### ^{∗}Example A23: Reaction using maleic acid

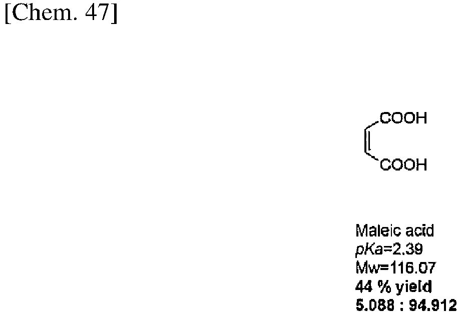

The reaction was carried out in accordance with General synthesis procedure A using maleic acid (pKa=2.39, Mw=116.07) as the acidic compound to thereby afford the target compound. Yield: 44%, dr = 94.9:5,1.

### ^{∗}Example A24: Reaction using citric acid

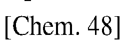

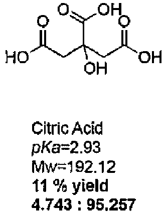

The reaction was carried out in accordance with General synthesis procedure A using citric acid (pKa=2.93, Mw=192.12) as the acidic compound to thereby afford the target compound. Yield: 11%, dr = 95.3:4.7.

### ^{∗}Example A25: Reaction using lactic acid methyl

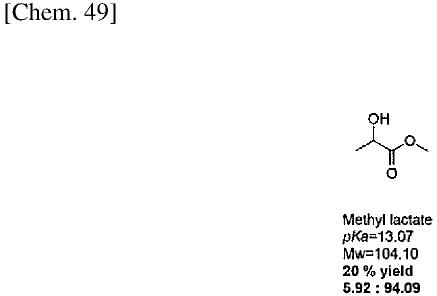

The reaction was carried out in accordance with General synthesis procedure A using lactic acid methyl (pKa=13.07, Mw=104.10) as the acidic compound to thereby afford the target compound. Yield: 20%, dr = 94.1:5.9.

### ^{∗}Example A26: Reaction using ethyl lactate

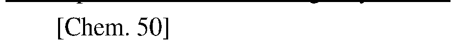

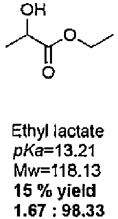

The reaction was carried out in accordance with General synthesis procedure A using ethyl lactate (pKa=13.21, Mw=118.13) as the acidic compound to thereby afford the target compound. Yield: 15%, dr = 98.3:1.7.

### ^{∗}Example A27: Reaction using trichloroacetic acid

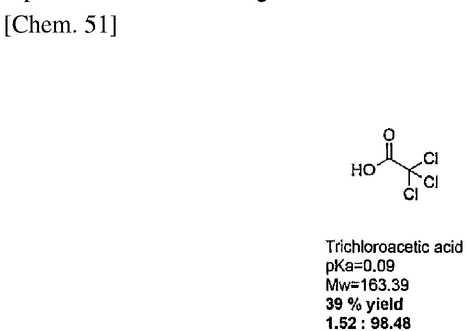

The reaction was carried out in accordance with General synthesis procedure A using trichloroacetic acid (pKa=0.09, Mw=163.39) as the acidic compound to thereby afford the target compound. Yield: 39%, dr = 98.5:1.5.

### ^{∗}Example A28: Reaction using bromoacetic acid

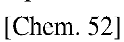

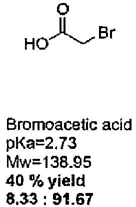

The reaction was carried out in accordance with General synthesis procedure A using bromoacetic acid (pKa=2.73, Mw=138.85) as the acidic compound to thereby afford the target compound. Yield: 40%, dr = 91.7:8.3.

### ^{∗}Example A29: Reaction using 1-adamantane carboxylic acid

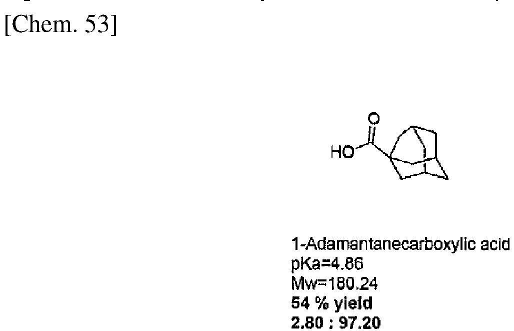

The reaction was carried out in accordance with General synthesis procedure A using 1-adamantane carboxylic acid (pKa=4.86, Mw=180.24) as the acidic compound to thereby afford the target compound. Yield: 54%, dr = 97.2:2.8.

### ^{∗}Example A30: Reaction using (+)-10-camphorsulfonic acid

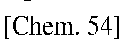

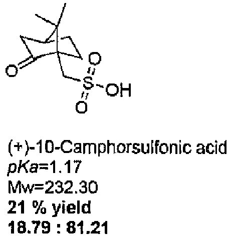

The reaction was carried out in accordance with General synthesis procedure A using (+)-10-camphorsulfonic acid (pKa=1.17, Mw=232.30) as the acidic compound to thereby afford the target compound. Yield: 21%, dr = 81.2:18.8.

### ^{∗}Example A31: Reaction using benzoic acid

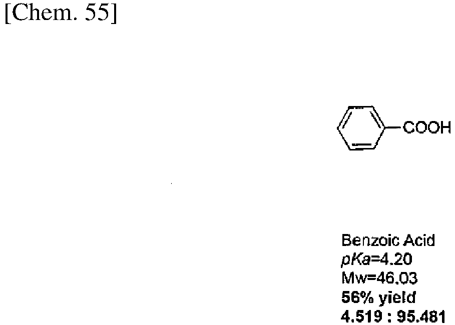

The reaction was carried out in accordance with General synthesis procedure A using benzoic acid (pKa=4.20, Mw=46.03) as the acidic compound to thereby afford the target compound. Yield: 56%, dr = 95.5:4.5.

### ^{∗}Example A32: Reaction using 2,6-dimethylbenzoic acid

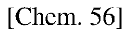

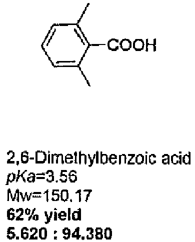

The reaction was carried out in accordance with General synthesis procedure A using 2,6-dimethylbenzoic acid (pKa=3.56, Mw=150.17) as the acidic compound to thereby afford the target compound. Yield: 62%, dr = 94.4:5.6.

### ^{∗}Example A33: Reaction using 2,4,6-trimethylbenzoic acid

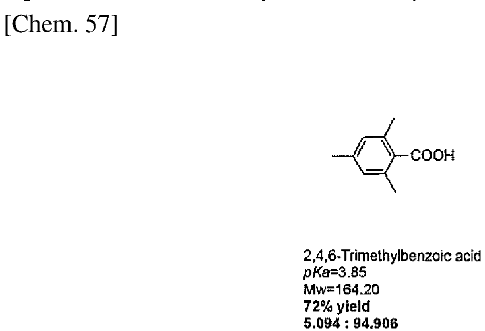

The reaction was carried out in accordance with General synthesis procedure A using 2,4,6-trimethylbenzoic acid (pKa=3.85, Mw=164.20) as the acidic compound to thereby afford the target compound. Yield: 72%, dr = 94.9:5.1.

### ^{∗}Example A34: Reaction using 2,6-diisopropylbenzoic acid

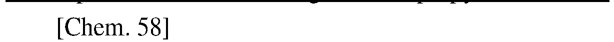

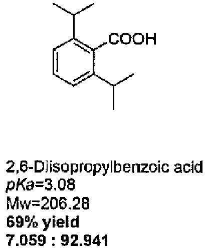

The reaction was carried out in accordance with General synthesis procedure A using 2,6-diisopropylbenzoic acid (pKa=3.08, Mw=206.28) as the acidic compound to thereby afford the target compound. Yield: 69%, dr = 92.9:7.1.

### ^{∗}Example A35: Reaction using 2,6-di-tert-butylbenzoic acid

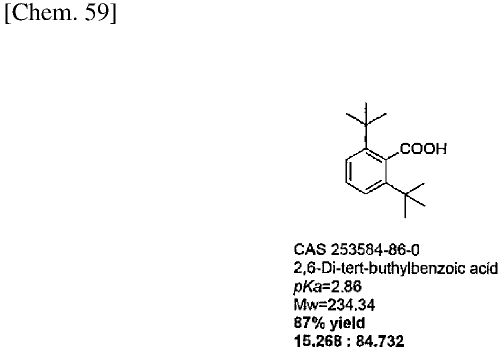

The reaction was carried out in accordance with General synthesis procedure A using 2,6-di-tert-butylbenzoic acid (pKa=2.86, Mw=234.34) as the acidic compound to thereby afford the target compound. Yield: 87%, dr = 84.7:15.3.

### ^{∗}Example A36: Reaction using 2,4,6-triisopropylbenzoic acid

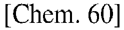

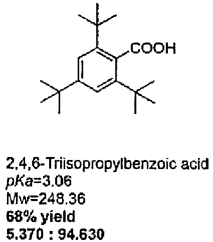

The reaction was carried out in accordance with General synthesis procedure A using 2,4,6-triisopropylbenzoic acid (pKa=3.06, Mw=248.36) as the acidic compound to thereby afford the target compound. Yield: 68%, dr = 94.6:5.4.

### ^{∗}Example A37: Reaction using 2,6-dimethoxy benzoic acid

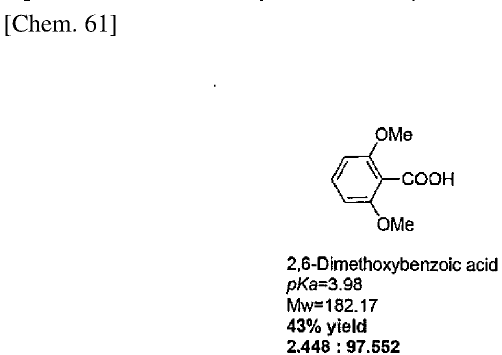

The reaction was carried out in accordance with General synthesis procedure A using 2,6-dimethoxy benzoic acid (pKa=3.98, Mw=182.17) as the acidic compound to thereby afford the target compound. Yield: 43%, dr = 97.6:2.4.

### ^{∗}Example A38: Reaction using phthalic acid

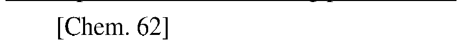

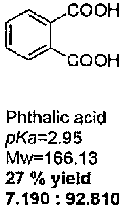

The reaction was carried out in accordance with General synthesis procedure A using phthalic acid (pKa=2.95, Mw=166.13) as the acidic compound to thereby afford the target compound. Yield: 27%, dr = 92.8:7.2.

### ^{∗}Example A39: Reaction using isophthalic acid

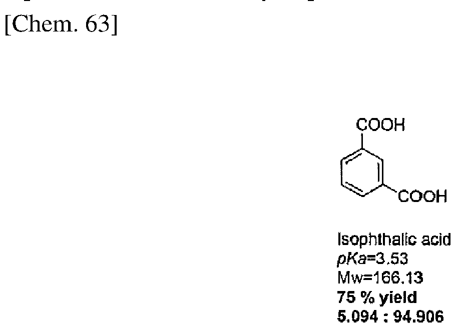

The reaction was carried out in accordance with General synthesis procedure A using isophthalic acid (pKa=3.53, Mw=166.13) as the acidic compound to thereby afford the target compound. Yield: 75%, dr = 94.9:5.1.

### ^{∗}Example A40: Reaction using 1,2-phenylene diacetate

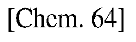

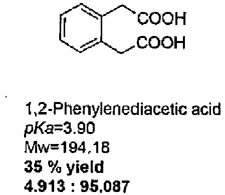

The reaction was carried out in accordance with General synthesis procedure A using 1,2-phenylene diacetate (pKa=3.90, Mw=194.18) as the acidic compound to thereby afford the target compound. Yield: 35%, dr = 95.1:4.9.

### ^{∗}Examole A41 Reaction using pyromellitic acid

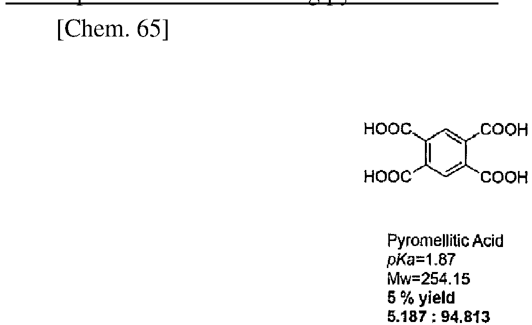

The reaction was carried out in accordance with General synthesis procedure A using pyromellitic acid (pKa=1.87, Mw=254.15) as the acidic compound to thereby afford the target compound. Yield: 5%, dr = 94.8:5.2.

### ^{∗}Example A42: Reaction using L-proline

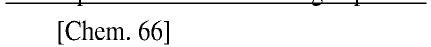

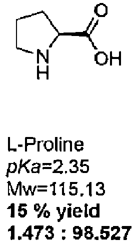

The reaction was carried out in accordance with General synthesis procedure A using L-proline (pKa=2.35, Mw=115.13) as the acidic compound to thereby afford the target compound. Yield: 15%, dr = 98.5:1.5.

### ^{∗}Example A43: Reaction using proline tetrazole

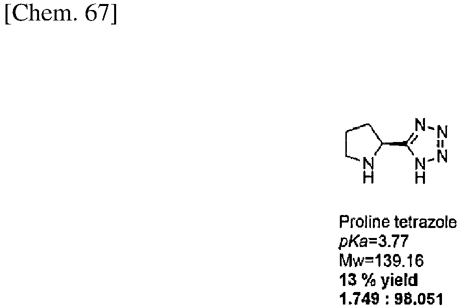

The reaction was carried out in accordance with General synthesis procedure A using proline tetrazole (pKa=3.77, Mw=139.16) as the acidic compound to thereby afford the target compound. Yield: 13%, dr = 98.1:1.7.

### ^{∗}Example A44: Reaction using (S)-2-(1H-tetrazol-5-yl)pyrrolidine-1-carboxylic acid tert-butyl ester

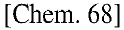

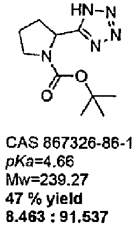

The reaction was carried out in accordance with General synthesis procedure A using (S)-2-(1H-tetrazol-5-yl)pyrrolidine-1-carboxylic acid tert-butyl ester (pKa=4.66, Mw=239.27) as the acidic compound to thereby afford the target compound. Yield: 47%, dr = 91.5:8.5.

### ^{∗}Example A45: Reaction using 1,1'-binaphthyl-2,2'-dicarboxylic acid

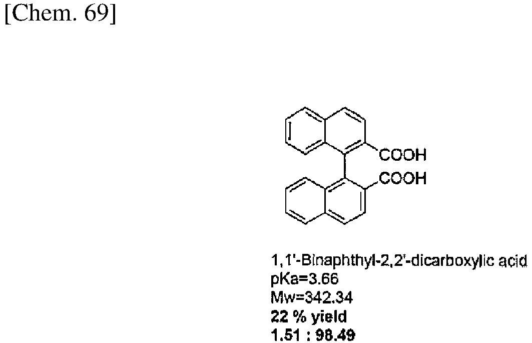

The reaction was carried out in accordance with General synthesis procedure A using 1,1'-binaphthyl-2,2'-dicarboxylic acid (pKa=3.66, Mw=342.34) as the acidic compound to thereby afford the target compound. Yield: 22%, dr = 98.5:1.5.

### ^{∗}Example A46: Reaction using phenol

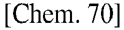

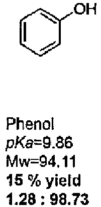

The reaction was carried out in accordance with General synthesis procedure A using phenol (pKa=9.86, Mw=94.11) as the acidic compound to thereby afford the target compound. Yield: 15%, dr = 98.7:1.3.

### ^{∗}Example A47: Reaction using benzenethiol

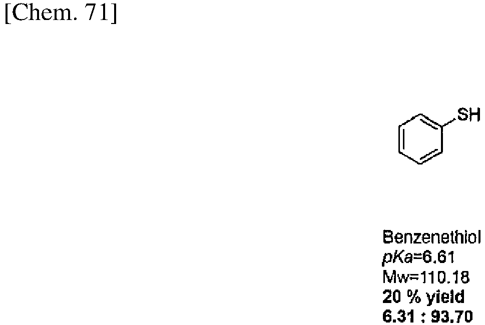

The reaction was carried out in accordance with General synthesis procedure A using benzenethiol (pKa=6.61, Mw=110.18) as the acidic compound to thereby afford the target compound. Yield: 20%, dr = 93.7:6.3.

### ^{∗}Example A48: Reaction using catechol

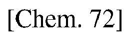

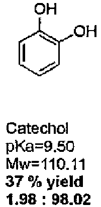

The reaction was carried out in accordance with General synthesis procedure A using catechol (pKa=9.50, Mw=110.11) as the acidic compound to thereby afford the target compound. Yield: 37%, dr = 98.0:2.0.

### ^{∗}Example A49: Reaction using 4-nitrophenol

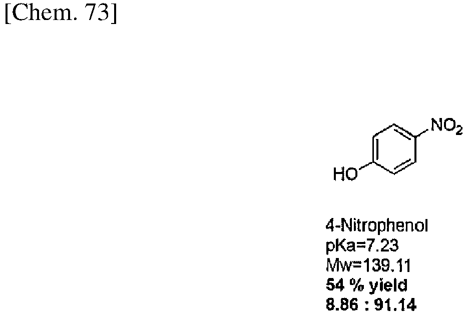

The reaction was carried out in accordance with General synthesis procedure A using 4-nitrophenol (pKa=7.23, Mw=139.11) as the acidic compound to thereby afford the target compound. Yield: 54%, dr = 91.1:8.9.

### ^{∗}Example A50: Reaction using 4-methoxyphenol

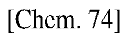

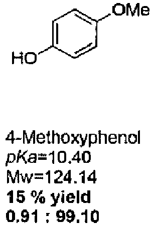

The reaction was carried out in accordance with General synthesis procedure A using 4-methoxyphenol (pKa=10.40, Mw=124.14) as the acidic compound to thereby afford the target compound. Yield: 15%, dr = 99.1:0.9.

### ^{∗}Example A51: Reaction using p-toluene boronic acid

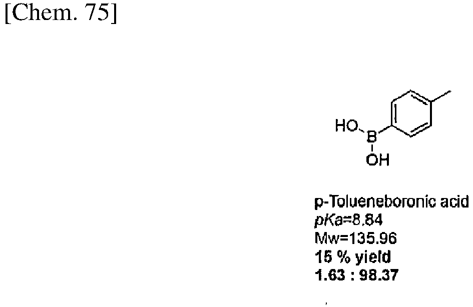

The reaction was carried out in accordance with General synthesis procedure A using p-toluene boronic acid (pKa=8.84, Mw=135.96) as the acidic compound to thereby afford the target compound. Yield: 15%, dr = 98.4:1.6.

### ^{∗}Example A52: Reaction using 4-methoxyphenylboronic acid

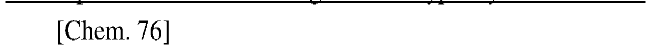

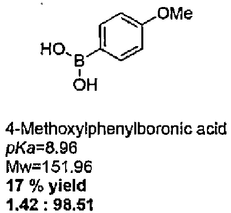

The reaction was carried out in accordance with General synthesis procedure A using 4-methoxyphenylboronic acid (pKa=8.96, Mw=151.96) as the acidic compound to thereby afford the target compound. Yield: 17%, dr = 98.5:1.4.

### ^{∗}Example A53: Reaction using 2,4,6-trimethylphenol

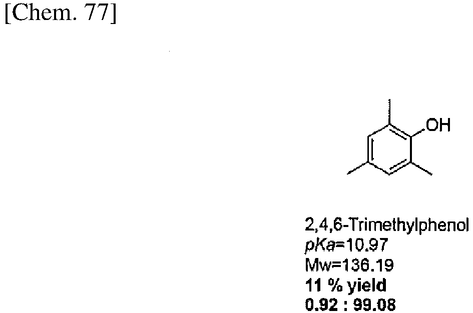

The reaction was carried out in accordance with General synthesis procedure A using 2,4,6-trimethylphenol (pKa=10.97, Mw=136.19) as the acidic compound to thereby afford the target compound. Yield: 11%, dr = 99.1:0.9.

### ^{∗}Example A54: Reaction using 2,6-di-tert-butyl-4-methylphenol

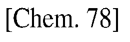

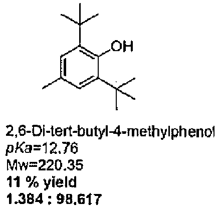

The reaction was carried out in accordance with General synthesis procedure A using 2,6-di-tert-butyl-4-methylphenol (pKa=12.76, Mw=220.35) as the acidic compound to thereby afford the target compound. Yield: 11%, dr = 98.6:1.4.

### ^{∗}Example A55: Reaction using N-(2,6-dimethylphenyl)-1,1,1-trifluoromethanesulfonamide

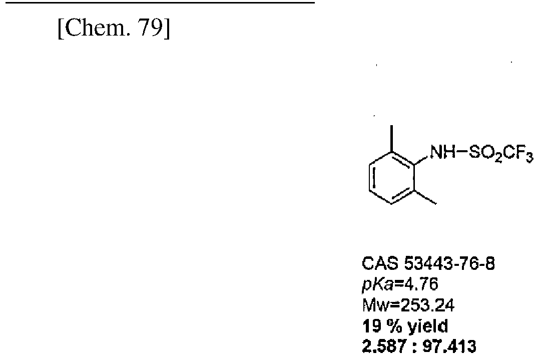

The reaction was carried out in accordance with General synthesis procedure A using N-(2,6-dimethylphenyl)-1,1,1-trifluoromethanesulfonamide (pKa=4.76, Mw=253.24) as the acidic compound to thereby afford the target compound. Yield: 19%, dr = 97.4:2.6.

### ^{∗}Example A56: Reaction using N-[2,6-bis(1-methylethyl)phenyl]-1,1,1-trifluoromethanesulfonamide

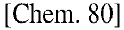

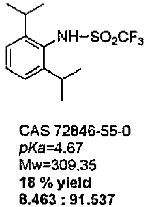

The reaction was carried out in accordance with General synthesis procedure A using N-[2,6-bis(1-methylethyl)phenyl]-1,1,1-trifluoromethanesulfonamide (pKa=4.67, Mw=309.35) as the acidic compound to thereby afford the target compound. Yield: 18%, dr = 91.5:8.5.

### ^{∗}Example A57: Reaction using 4-methylphenylsulfonic acid anhydride

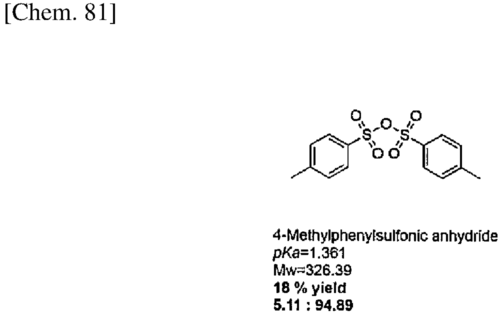

The reaction was carried out in accordance with General synthesis procedure A using 4-methylphenylsulfonic acid anhydride (pKa=1.361, Mw=326.39) as the acidic compound to thereby afford the target compound. Yield: 18%, dr = 94.9:5.1.

### ^{∗}Example A58: Reaction using methanesulfonic acid

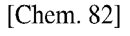

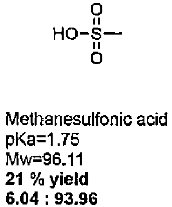

The reaction was carried out in accordance with General synthesis procedure A using methanesulfonic acid (pKa=1.75, Mw=96.11) as the acidic compound to thereby afford the target compound. Yield: 21%, dr = 94.0:6.0.

### ^{∗}Example A59: Reaction using tert-butyldimethylsilanol

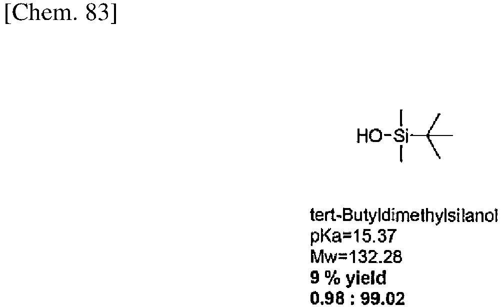

The reaction was carried out in accordance with General synthesis procedure A using tert-butyldimethylsilanol (pKa=15.37, Mw=132.28) as the acidic compound to thereby afford the target compound. Yield: 9%, dr = 99.0:1.0.

### ^{∗}Example A60: Reaction using triphenylsilanol

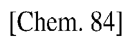

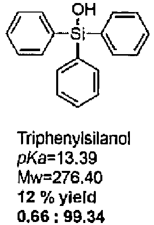

The reaction was carried out in accordance with General synthesis procedure A using triphenylsilanol (pKa=13.39, Mw=276.40) as the acidic compound to thereby afford the target compound. Yield: 12%, dr = 99.3:0.7.

### ^{∗}Example A61: Reaction using 2,2,2-trifluoroethanol

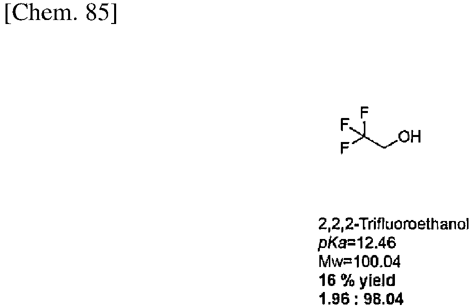

The reaction was carried out in accordance with General synthesis procedure A using 2,2,2-trifluoroethanol (pKa=12.46, Mw=100.04) as the acidic compound to thereby afford the target compound. Yield: 16%, dr = 98.0:2.0.

### [Example Group B: Amidation reactions using various Reactant Compound 11

### ^{∗}General synthesis procedure B:

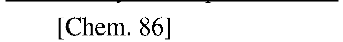

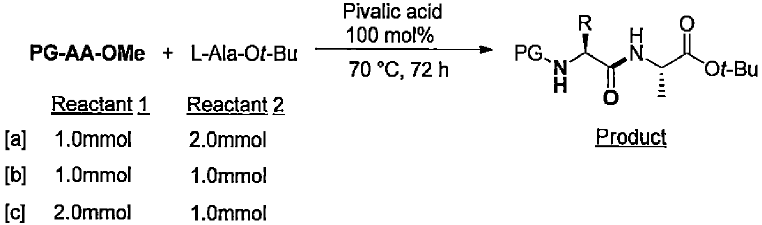

Under argon atmosphere, in a glove box, a heat-dried 5.0-mL screw-cap vial containing a stirrer (samarium-cobalt) was charged with PG-AA-OMe (where AA refers to an amino acid and PG refers to a protective group of the amino group of the amino acid AA) as Reactant Compound 1 and L-Ala-Ot-Bu as Reactant Compound 2 at ratios satisfying any one of Conditions [a] to [c] below. In addition, pivalic acid (102.1mg, 1.0 mmol) is added as the Brønsted acid, the reaction mixture was stirred vigorously at 70°C for 72 hours in a preheated oil bath under nitrogen atmosphere, then the reaction was cooled to ambient temperature. The reaction mixture was diluted with CHCl₃ (3.0 mL) and pipetted into a transferred to a silica gel column, and the used vial and pipette were washed with CHCl₃ (12.0 mL). The used vial and pipette were washed with CHCl₃ (12.0 mL). The reaction mixture was purified by flash column chromatography (using 0 to 60% AcOEt in hexane as mobile phase) to thereby afford the title compound of each Example. ^{∗}Condition [a]: Reactant Compound 1 = 1.0mmol, Reactant Compound 2 = 2.0mmol. ^{∗}Condition [b]: Reactant Compound 1 = 1.0mmol, Reactant Compound 2 = 1.0mmol. ^{∗}Condition [c]: Reactant Compound 1 = 2.0mmol, Reactant Compound 2 = 1.0mmol.

Among the values of yield, er, and dr in this Example Group, the values marked with ^{∗} were measured by HPLC analysis, and those marked with ^{∗∗} were by ¹H-NMR analysis.

### ^{∗}Example B1: Synthesis of Boc-Gly-L-Ala-Ot-Bu

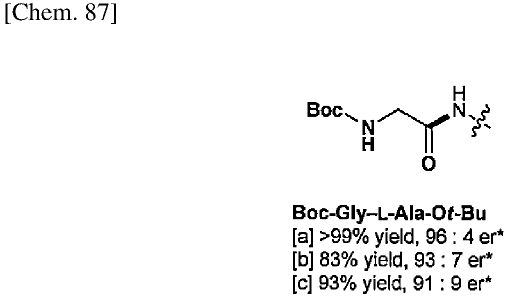

The reaction was carried out in accordance with General synthesis procedure B using Boc-Gly-OMe as Reactant Compound 1 to thereby afford the title compound Boc-Gly-L-Ala-Ot-Bu as colorless liquid.
^{∗}Condition [a]: Yield > 99%, er=96: 4.
^{∗}Condition [b]: Yield 83%, er=93: 7.
^{∗}Condition [c]: Yield 93%, er=91: 9.

### ^{∗}Example B2: Synthesis of Boc-L-Ala-L-Ala-Ot-Bu

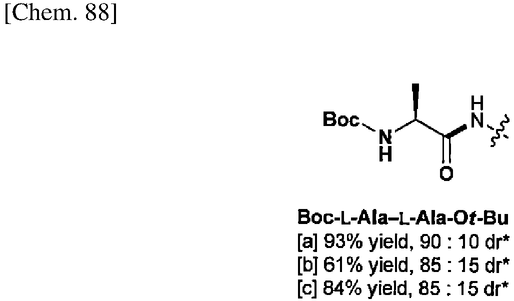

The reaction was carried out in accordance with General synthesis procedure B using Boc-L-Ala-OMe as Reactant Compound 1 to thereby afford the title compound Boc-L-Ala-L-Ala-Ot-Bu as colorless liquid.
^{∗}Condition [a]: Yield 93%, dr = 90:10.
^{∗}Condition [b]: Yield 61%, dr = 85:15.
^{∗}Condition [c]: Yield 84%, dr = 85:15.

### ^{∗}Example B3: Synthesis of Boc-L-Leu-L-Ala-Ot-Bu

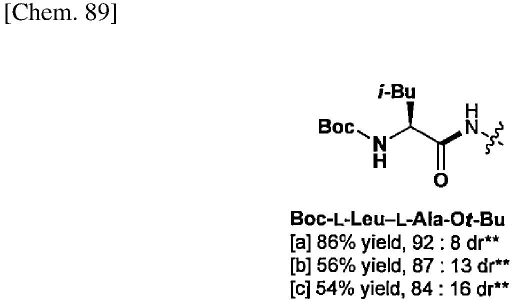

The reaction was carried out in accordance with General synthesis procedure B using Boc-L-Leu-OMe as Reactant Compound 1 to thereby afford the title compound Boc-L-Leu-L-Ala-Ot-Bu as white solid.
^{∗}Condition [a]: Yield 86%, dr = 92: 8.
^{∗}Condition [b]: Yield 56%, dr = 87:13.
^{∗}Condition [c]: Yield 54%, dr = 84:16.

### ^{∗}Example B3': Synthesis of Cbz-L-Leu-L-Ala-Ot-Bu

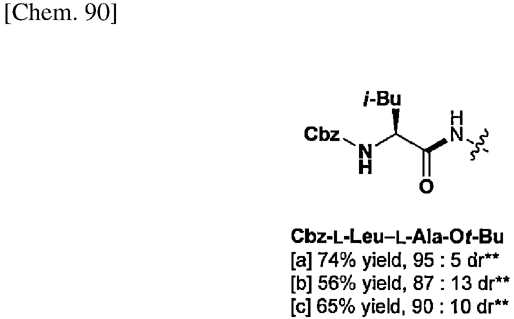

The reaction was carried out in accordance with General synthesis procedure B using Cbz-L-Leu-OMe as Reactant Compound 1 to thereby afford the title compound Cbz-L-Leu-L-Ala-Ot-Bu as white solid.
^{∗}Condition [a]: Yield 74%, dr = 95: 5.
^{∗}Condition [b]: Yield 56%, dr = 87:13.
^{∗}Condition [c]: Yield 65%, dr = 90:10.

### ^{∗}Example B4: Synthesis of Boc-L-Ile-L-Ala-Ot-Bu

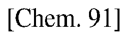

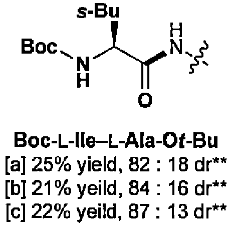

The reaction was carried out in accordance with General synthesis procedure B using Boc-L-Ile-OMe as Reactant Compound 1 to thereby afford the title compound Boc-L-Ile-L-Ala-Ot-Bu as white solid.
^{∗}Condition [a]: Yield 25%, dr = 82:18.
^{∗}Condition [b]: Yield 21%, dr = 84:16.
^{∗}Condition [c]: Yield 22%, dr = 87:13.

### ^{∗}Example B5: Synthesis of Boc-L-Val-L-Ala-Ot-Bu

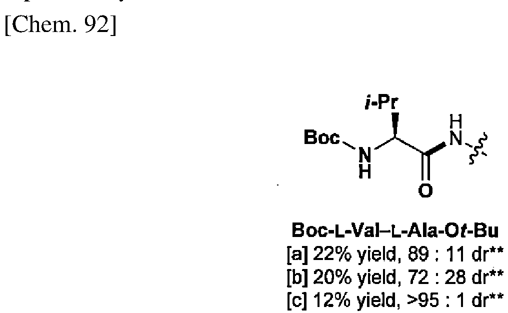

The reaction was carried out in accordance with General synthesis procedure B using Boc-L-Val-OMe as Reactant Compound 1 to thereby afford the title compound Boc-L-Val-L-Ala-Ot-Bu as white solid.
^{∗}Condition [a]: Yield 22%, dr = 89:11.
^{∗}Condition [b]: Yield 20%, dr = 72:28.
^{∗}Condition [c]: Yield 12%, dr = >95: 1.

### ^{∗}Example B6: Synthesis of Boc-L-Phe-L-Ala-Ot-Bu

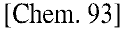

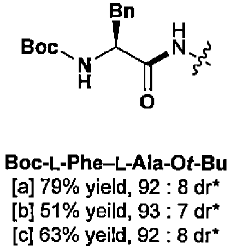

The reaction was carried out in accordance with General synthesis procedure B using Boc-L-Phe-OMe as Reactant Compound 1 to thereby afford the title compound Boc-L-Phe-L-Ala-Ot-Bu as white solid.
^{∗}Condition [a]: Yield 79%, dr = 92: 8.
^{∗}Condition [b]: Yield 51%, dr = 93: 7.
^{∗}Condition [c]: Yield 63%, dr = 92: 8.

### ^{∗}Example B7: Synthesis of Boc-L-Trp-L-Ala-Ot-Bu

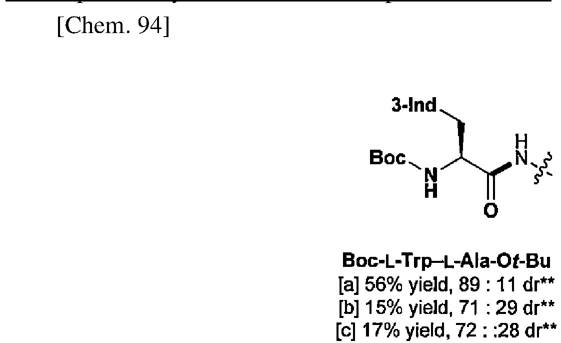

The reaction was carried out in accordance with General synthesis procedure B using Boc-L-Trp-OMe as Reactant Compound 1 to thereby afford the title compound Boc-L-Trp-L-Ala-Ot-Bu as colorless liquid.
^{∗}Condition [a]: Yield 56%, dr = 89:11.
^{∗}Condition [b]: Yield 15%, dr = 71:29.
^{∗}Condition [c]: Yield 17%, dr = 72:28.

### ^{∗}Example B8: Synthesis of Boc-L-Thr-L-Ala-Ot-Bu

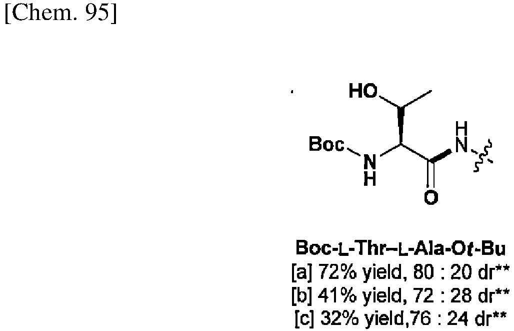

The reaction was carried out in accordance with General synthesis procedure B using Boc-L-Thr-OMe as Reactant Compound 1 to thereby afford the title compound Boc-L-Thr-L-Ala-Ot-Bu as colorless liquid.
^{∗}Condition [a]: Yield 72%, dr = 80:20.
^{∗}Condition [b]: Yield 41%, dr = 72:28.
^{∗}Condition [c]: Yield 32%, dr = 76:24.

### ^{∗}Example B9: Synthesis of Cbz-L-Met-L-Ala-Ot-Bu

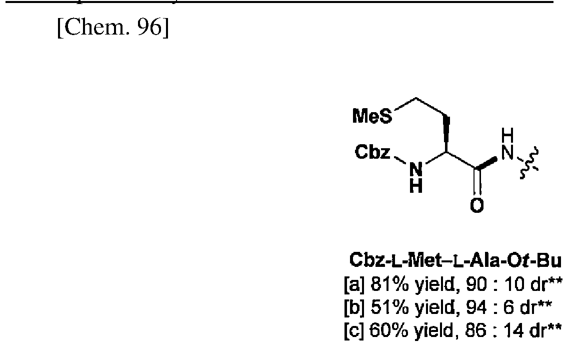

The reaction was carried out in accordance with General synthesis procedure B using Cbz-L-Met-OMe as Reactant Compound 1 to thereby afford the title compound Cbz-L-Met-L-Ala-Ot-Bu as white solid.
^{∗}Condition [a]: Yield 81%, dr = 90:10.
^{∗}Condition [b]: Yield 51%, dr = 94: 6.
^{∗}Condition [c]: Yield 60%, dr = 86:14.

### ^{∗}Example B10: Synthesis of Boc-L-Pro-L-Ala-Ot-Bu

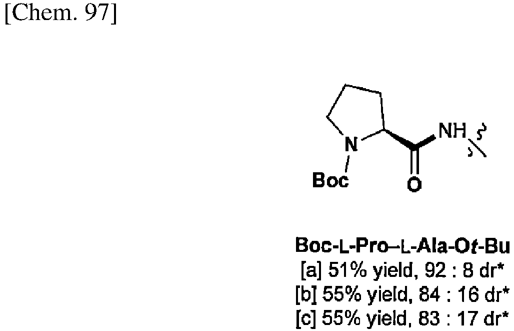

The reaction was carried out in accordance with General synthesis procedure B using Boc-L-Pro-OMe as Reactant Compound 1 to thereby afford the title compound Boc-L-Pro-L-Ala-Ot-Bu as white solid.
^{∗}Condition [a]: Yield 51%, dr = 92: 8.
^{∗}Condition [b]: Yield 55%, dr = 84:16.
^{∗}Condition [c]: Yield 55%, dr = 83:17.

### [Example Group C: Amidation reactions various Reactant Compounds 21

### ^{∗}General synthesis procedure C:

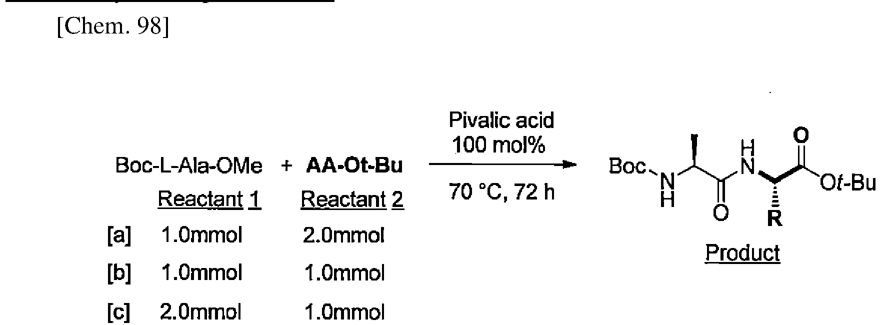

Under argon atmosphere, in a glove box, a heat-dried 5.0-mL screw-cap vial containing a stirrer (samarium-cobalt) was charged with Boc-L-Ala-Ot-Bu as Reactant Compound 1 and AA-Ot-Bu (where AA refers to an amino acid) as Reactant Compound 2 at ratios satisfying any one of Conditions [a] to [c] below. In addition, pivalic acid (102.1mg, 1.0mmol) was added as the Brønsted acid, and the reaction mixture was stirred vigorously at 70°C for 72 hours in a preheated oil bath under nitrogen atmosphere, then the reaction was cooled to ambient temperature. The reaction mixture was diluted with CHCl₃ (3.0 mL) and pipetted into a transferred to a silica gel column, and the used vial and pipette were washed with CHCl₃ (12.0 mL). The used vial and pipette were washed with CHCl₃ (12.0 mL). The reaction mixture was purified by flash column chromatography (using 0 to 60% AcOEt in hexane as mobile phase) to thereby afford the title compound of each Example.
^{∗}Condition [a]: Reactant Compound 1 = 1.0mmol, Reactant Compound 2 = 2.0mmol.
^{∗}Condition [b]: Reactant Compound 1 = 1.0mmol, Reactant Compound 2 = 1.0mmol.
^{∗}Condition [c]: Reactant Compound 1 = 2.0mmol, Reactant Compound 2 = 1.0mmol.

Among the values of yield, er, and dr in this Example Group, the values marked with ^{∗} were measured by HPLC analysis, and those marked with ^{∗∗} were by ¹H-NMR analysis.

### ^{∗}Example C1: Synthesis of Boc-L-Ala-Gly-Ot-Bu Synthesis of

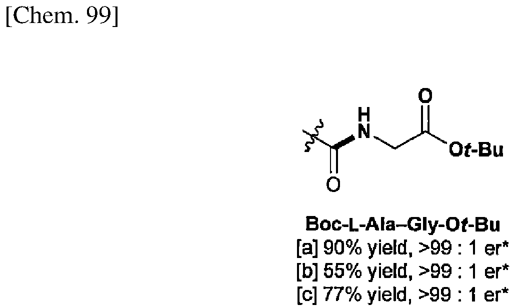

The reaction was carried out in accordance with General synthesis procedure C using Gly-Ot-Bu as Reactant Compound 2 to thereby afford the title compound Boc-L-Ala-Gly-Ot-Bu as colorless liquid.
^{∗}Condition [a]: Yield 90%, er=>99: 1.
^{∗}Condition [b]: Yield 55%, er=>99: 1.
^{∗}Condition [c]: Yield 77%, er=>99: 1.

### ^{∗}Example C2: Synthesis of Boc-L-Ala-L-Ala-Ot-Bu

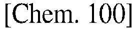

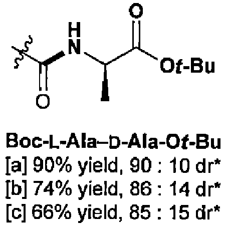

The reaction was carried out in accordance with General synthesis procedure C using L-Ala-Ot-Bu as Reactant Compound 2 to thereby afford the title compound Boc-L-Ala-L-Ala-Ot-Bu as white solid.
^{∗}Condition [a]: Yield 90%, dr = 90:10.
^{∗}Condition [b]: Yield 74%, dr = 86:14.
^{∗}Condition [c]: Yield 66%, dr = 85:15.

### ^{∗}Example C3: Synthesis of Boc-L-Ala-L-Leu-Ot-Bu

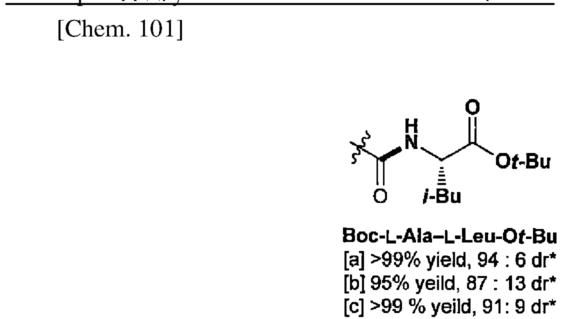

The reaction was carried out in accordance with General synthesis procedure C using L-Leu-Ot-Bu as Reactant Compound 2 to thereby afford the title compound Boc-L-Ala-L-Leu-Ot-Bu as colorless liquid.
^{∗}Condition [a]: Yield >99%, dr = 94: 6.
^{∗}Condition [b]: Yield 95%, dr = 87:13.
^{∗}Condition [c]: Yield >99%, dr = 91: 9.

### ^{∗}Example C4: Synthesis of Boc-L-Ala-L-Ile-Ot-Bu

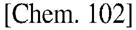

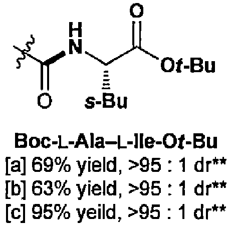

The reaction was carried out in accordance with General synthesis procedure C using L-Ile-Ot-Bu as Reactant Compound 2 to thereby afford the title compound Boc-L-Ala-L-Ile-Ot-Bu as colorless liquid.
^{∗}Condition [a]: Yield 69%, dr = >95: 1.
^{∗}Condition [b]: Yield 63%, dr = >95: 1.
^{∗}Condition [c]: Yield 95%, dr = >95: 1.

### ^{∗}Example C5: Synthesis of Boc-L-Ala-L-Val-Ot-Bu

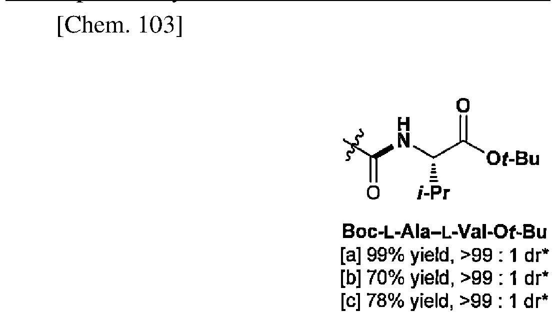

The reaction was carried out in accordance with General synthesis procedure C using L-Val-Ot-Bu as Reactant Compound 2 to thereby afford the title compound Boc-L-Ala-L-Val-Ot-Bu as colorless liquid.
^{∗}Condition [a]: Yield 99%, dr = >99: 1.
^{∗}Condition [b]: Yield 70%, dr = >99: 1.
^{∗}Condition [c]: Yield 78%, dr = >99: 1.

### ^{∗}Example C5': Synthesis of Boc-L-Ala-D-Val-Ot-Bu

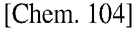

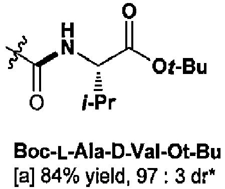

The reaction was carried out in accordance with General synthesis procedure C using D-Val-Ot-Bu as Reactant Compound 2 to thereby afford the title compound Boc-L-Ala-D-Val-Ot-Bu as colorless liquid.
^{∗}Condition [a]: Yield 84%, dr = 97: 3.

### ^{∗}Example C6: Synthesis of Boc-L-Ala-L-Ph-Ot-Bu

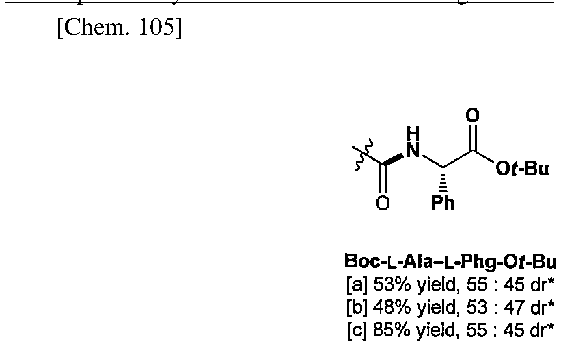

The reaction was carried out in accordance with General synthesis procedure C using L-Phg-Ot-Bu as Reactant Compound 2 to thereby afford the title compound Boc-L-Ala-L-Phg-Ot-Bu as white solid.
^{∗}Condition [a]: Yield 53%, dr = 55:45.
^{∗}Condition [b]: Yield 48%, dr = 53:47.
^{∗}Condition [c]: Yield 85%, dr = 55:45.

### ^{∗}Example C7: Synthesis of Boc-L-Ala-L-Phe-Ot-Bu

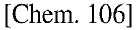

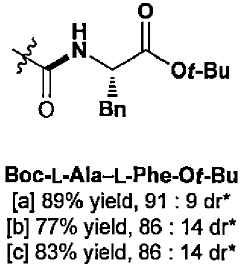

The reaction was carried out in accordance with General synthesis procedure C using L-Phe-Ot-Bu as Reactant Compound 2 to thereby afford the title compound Boc-L-Ala-L-Phe-Ot-Bu as white solid.
^{∗}Condition [a]: Yield 89%, dr = 91: 9.
^{∗}Condition [b]: Yield 77%, dr = 86:14.
^{∗}Condition [c]: Yield 83%, dr = 86:14.

### ^{∗}Example C8: Synthesis of Boc-L-Ala-L-Tr-Ot-Bu

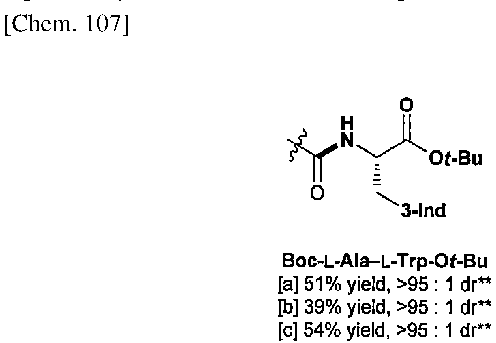

The reaction was carried out in accordance with General synthesis procedure C using L-Trp-Ot-Bu as Reactant Compound 2 to thereby afford the title compound Boc-L-Ala-L-Trp-Ot-Bu as colorless liquid.
^{∗}Condition [a]: Yield 51%, dr = >95: 1.
^{∗}Condition [b]: Yield 39%, dr = >95: 1.
^{∗}Condition [c]: Yield 54%, dr = >95: 1.

### ^{∗}Example C9: Synthesis of Boc-L-Ala-L-Thr(t-Bu)-Ot-Bu

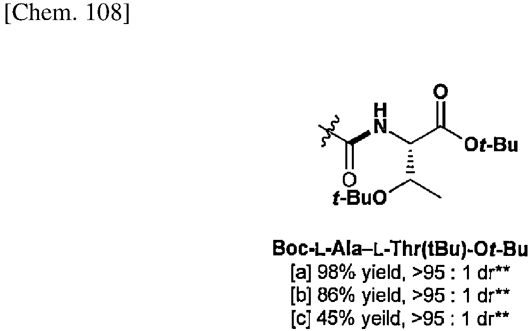

The reaction was carried out in accordance with General synthesis procedure C using L-Thr(t-Bu)-Ot-Bu as Reactant Compound 2 to thereby afford the title compound Boc-L-Ala-L-Thr(t-Bu)-Ot-Bu as colorless liquid.
^{∗}Condition [a]: Yield 98%, dr = >95: 1.
^{∗}Condition [b]: Yield 86%, dr = >95: 1.
^{∗}Condition [c]: Yield 45%, dr = >95: 1.

### ^{∗}Example C10: Synthesis of Boc-L-Ala-L-Met-Ot-Bu

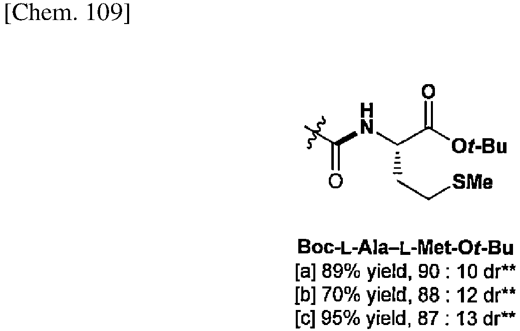

The reaction was carried out in accordance with General synthesis procedure C using L-Met-Ot-Bu as Reactant Compound 2 to thereby afford the title compound Boc-L-Ala-L-Met-Ot-Bu as colorless liquid.
^{∗}Condition [a]: Yield 89%, dr = 90:10.
^{∗}Condition [b]: Yield 70%, dr = 88:12.
^{∗}Condition [c]: Yield 95%, dr = 87:13.

### ^{∗}Example C11: Synthesis of Boc-L-Ala-L-Lys(Cbz)-Ot-Bu

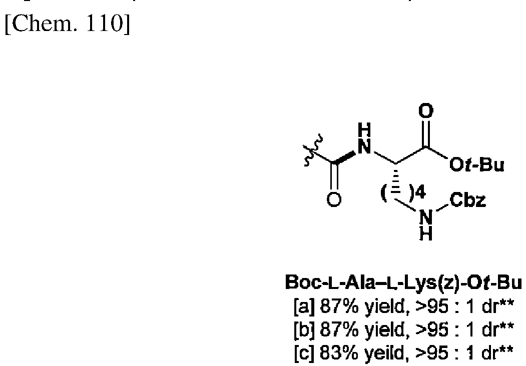

The reaction was carried out in accordance with General synthesis procedure C using L-Lys(Cbz)-Ot-Bu as Reactant Compound 2 to thereby afford the title compound Boc-L-Ala-L-Lys(Cbz)-Ot-Bu as white solid.
^{∗}Condition [a]: Yield 87%, dr = >95: 1.
^{∗}Condition [b]: Yield 87%, dr = >95: 1.
^{∗}Condition [c]: Yield 83%, dr = >95: 1.

### ^{∗}Example C12: Synthesis of Boc-L-Ala-L-Pro-Ot-Bu

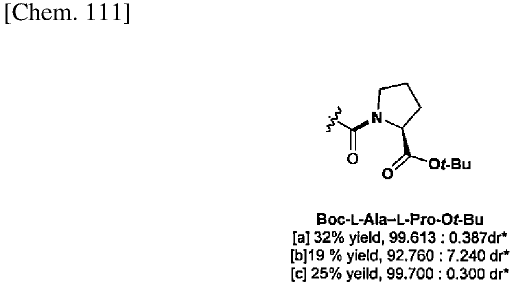

The reaction was carried out in accordance with General synthesis procedure C using L-Pro-Ot-Bu as Reactant Compound 2 to thereby afford the title compound Boc-L-Ala-L-Pro-Ot-Bu as colorless liquid.
^{∗}Condition [a]: Yield 32%, dr = 99:1.
^{∗}Condition [b]: Yield 19%, dr = 93:7.
^{∗}Condition [c]: Yield 25%, dr = 99:1.

### [Example Group D: Amidation reactions using Reactant Compounds 1 having various protective groups PGs]

### ^{∗}General synthesis procedure D:

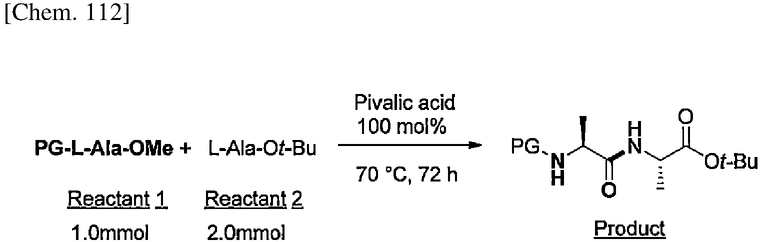

Under argon atmosphere, in a glove box, a heat-dried 5.0-mL screw-cap vial containing a stirrer (samarium-cobalt) was charged with PG-L-Ala-OMe (where PG refers to a protective group of the amino group of L-Ala) as Reactant Compound 1 and L-Ala-Ot-Bu as Reactant Compound 2 at ratios of Reactant Compound 1 = 1.0 mmol and Reactant Compound 2 = 2.0 mmol. In addition, pivalic acid was added as the Brønsted acid at a ratio satisfying any one of Condition [a] or [b], and the reaction mixture was stirred vigorously at 70°C for 72 hours in a preheated oil bath under nitrogen atmosphere, then the reaction was cooled to ambient temperature. The reaction mixture was diluted with CHCl₃ (3.0 mL) and pipetted into a transferred to a silica gel column, and the used vial and pipette were washed with CHCl₃ (12.0 mL). The used vial and pipette were washed with CHCl₃ (12.0 mL). The reaction mixture was purified by flash column chromatography (using 0 to 60% AcOEt in hexane as mobile phase) to thereby afford the title compound of each Example.
^{∗}Condition [a]: pivalic acid = 100 mol%.
^{∗}Condition [b]: pivalic acid = 50 mol%.

### ^{∗}Example D1: Synthesis of Boc-L-Ala-L-Ala-Ot-Bu

The reaction was carried out in accordance with General synthesis procedure C using Boc-L-Ala-OMe as Reactant Compound 1 to thereby afford the title compound Boc-L-Ala-L-Ala-Ot-Bu as colorless liquid.
^{∗}Condition [a]: Yield >99%, dr = 95: 5.
^{∗}Condition [b]: Yield >99%, dr = 95: 5.

### ^{∗}Example D2: Synthesis of Cbz-L-Ala-L-Ala-Ot-Bu

The reaction was carried out in accordance with General synthesis procedure C using Cbz-L-Ala-OMe as Reactant Compound 1 to thereby afford the title compound Cbz-L-Ala-L-Ala-Ot-Bu as white solid.
^{∗}Condition [a]: Yield 83%, dr = 93: 7.
^{∗}Condition [b]: Yield 83%, dr = 95: 5.

### ^{∗}Example D3: Synthesis of Bz-L-Ala-L-Ala-Ot-Bu

The reaction was carried out in accordance with General synthesis procedure C using Bz-L-Ala-OMe as Reactant Compound 1 to thereby afford the title compound Bz-L-Ala-L-Ala-Ot-Bu as white solid.
^{∗}Condition [a]: Yield 95%, dr = 95: 5.
^{∗}Condition [b]: Yield 97%, dr = 95: 5.

### ^{∗}Example D4: Synthesis of Bn-L-Ala-L-Ala-Ot-Bu

The reaction was carried out in accordance with General synthesis procedure C using Bn-L-Ala-OMe as Reactant Compound 1 to thereby afford the title compound Bn-L-Ala-L-Ala-Ot-Bu as white solid.
^{∗}Condition [a]: Yield 83%, dr = >95: 5.
^{∗}Condition [b]: Yield 83%, dr = >95: 5.

### ^{∗}Example D5: Synthesis of H-L-Ala-L-Ala-Ot-Bu

The reaction was carried out in accordance with General synthesis procedure C using H-L-Ala-OMe with no protective group as Reactant Compound 1 to thereby afford the title compound H-L-Ala-L-Ala-Ot-Bu as colorless liquid.
^{∗}Condition [a]: Yield 16%, dr = >95: 5.

### [Example Group E: Amidation reactions using Reactant Compounds 1 having carboxylic acid groups esterified with aromatic or heterocyclic moiety]

### ^{∗}Example E1: Synthesis of Boc-L-Ala-L-Ala-Ot-Bu via Boc-Ala-OPh

### (Step E1-1) Synthesis of Boc-Ala-OPh

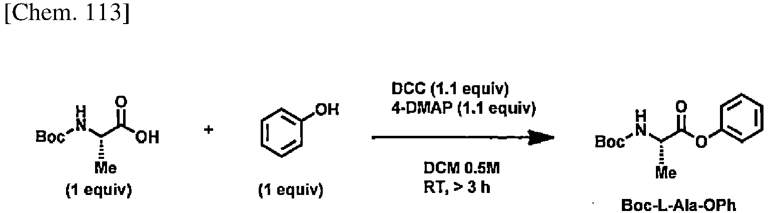

Under argon atmosphere, in a glove box, a heat-dried 5.0-mL screw-cap vial containing a stirrer (samarium-cobalt) was charged with Boc-L-Ala-OH (CAS No.: 15761-38-3, 946.1mg, 5.0mmol) and Ph-OH (470mg, 5.0mmol). This mixture was then combined with N,N'-dicyclohexyl carbodiimide (DCC) (1.135g, 5.5mmol) and N,N-dimethyl-4-amino pyridine (4-DMAP) (670mg, 5.5mmol), and then dissolved into dried DCM(10mL, 0.5M). The reaction mixture was stirred vigorously at room temperatures for 3 hours under argon atmosphere. The reaction was monitored by TLC analysis, and upon completion of the reaction, the reaction mixture was diluted with DCM, and then purified by flash column chromatography (using 0 to 30% AcOEt in hexane as mobile phase) to thereby afford the title compound Boc-Ala-OPh as white solid.

### (Step E1-2) Synthesis of Boc-L-Ala-L-Ala-Ot-Bu from Boc-Ala-OPh

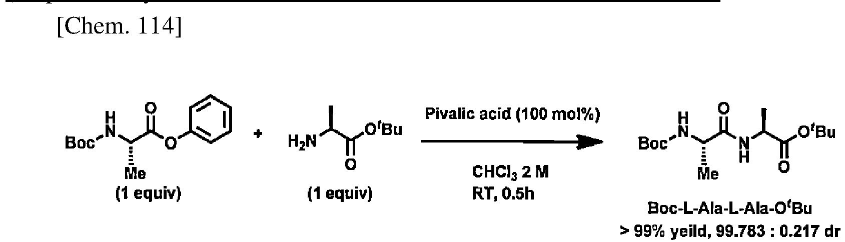

Under argon atmosphere, in a glove box, a heat-dried 5.0-mL screw-cap vial containing a stirrer (samarium-cobalt) was charged with Boc-L-Ala-OPh prepared in (Step E-1) (265.0mg, 1.0mmol) as Reactant Compound 1, L-Ala-Ot-Bu (290.4mg, 2.0mmol) as Reactant Compound 2, and pivalic acid (102.1mg, 1.0mmol) as the Brønsted acid, and the mixture was dissolved into dried CHCl₃ (0.5mL, 2M). The resulting mixture was stirred vigorously at room temperatures for 0.5 hours under nitrogen atmosphere. The reaction mixture was diluted with CHCl₃ (3.0 mL) and pipetted into a transferred to a silica gel column, and the used vial and pipette were washed with CHCl₃ (12.0 mL). The used vial and pipette were washed with CHCl₃ (12.0 mL). The reaction mixture was then purified by flash column chromatography (using 0 to 60% AcOEt in hexane as mobile phase) to thereby afford the title compound Boc-L-Ala-L-Ala-Ot-Bu as colorless liquid. Yield: >99%, dr >99:1.

### ^{∗}Example E2: Synthesis of Boc-L-Ala-L-Ala-Ot-Bu via Boc-Ala-ONp

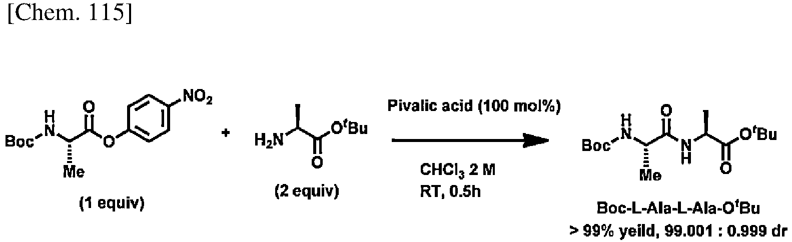

The reaction was carried out in accordance with the procedure mentioned above except that Boc-L-Ala-OPh was changed to Boc-L-Ala-ONp (CAS No. 2483-49-0, 310.3mg, 1.0mmol) to thereby afford the title compound Boc-L-Ala-L-Ala-Ot-Bu as colorless liquid. Yield: >99%, dr >99:1.

### ^{∗}Example E3: Synthesis of Boc-L-Ala-L-Ala-Ot-Bu via Boc-Ala-OSu

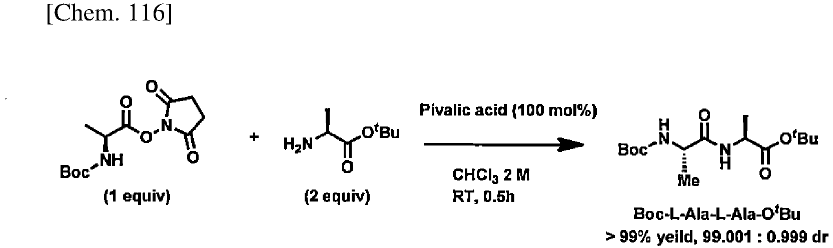

The reaction was carried out in accordance with the procedure mentioned above except that Boc-L-Ala-OPh was changed to Boc-L-Ala-OSu (CAS No. 3392-05-0, 286.3mg, 1.0mmol) to thereby afford the title compound Boc-L-Ala-L-Ala-Ot-Bu as colorless liquid. Yield: >99%, dr >99:1.

### ^{∗}Example E4: Synthesis of Boc-L-Ala-L-Phg-Ot-Bu via Boc-Ala-OPh

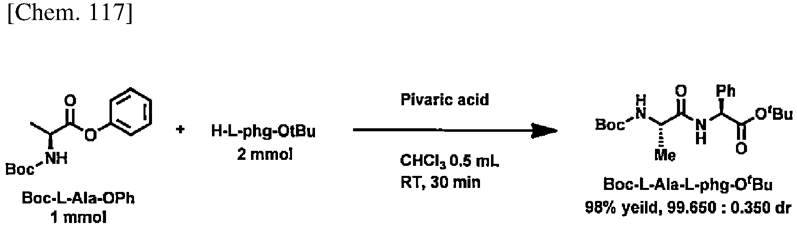

The reaction was carried out in accordance with the procedure mentioned above except that L-Ala-Ot-Bu was changed to L-Phg-Ot-Bu (CAS No. 53934-78-4, 414.6mg, 2.0mmol) to thereby afford the title compound Boc-L-Ala-L-Phg-Ot-Bu as white solid. Yield: 98%, dr >99:1.

### ^{∗}Example E5: Synthesis of Boc-L-Ala-L-Phg-Ot-Bu via Boc-Ala-OPh

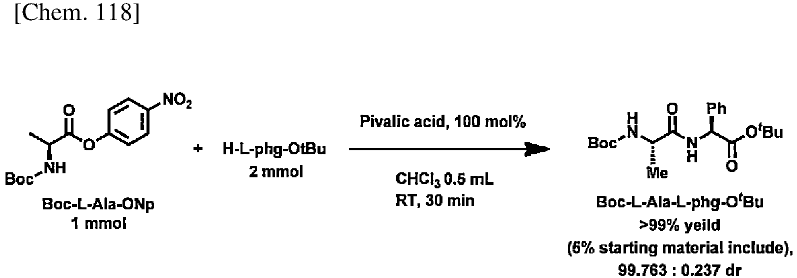

The reaction was carried out in accordance with the procedure mentioned above except that Boc-L-Ala-OPh was changed to Boc-L-Ala-ONp (CAS No. 2483-49-0, 310.3mg, 1.0mmol) and L-Ala-Ot-Bu was changed to L-Phg-Ot-Bu (CAS No. 53934-78-4, 414mg, 2.0mmol) to thereby afford the title compound Boc-L-Ala-L-Phg-Ot-Bu as white solid. Yield: >99%, dr >99:1.

### ^{∗}Example E6: Synthesis of Cbz-L-Ala-L-Ala-Ot-Bu via Cbz-Ala-OPh

### (Step E6-1) Synthesis of Cbz-Ala-OPh

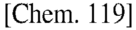

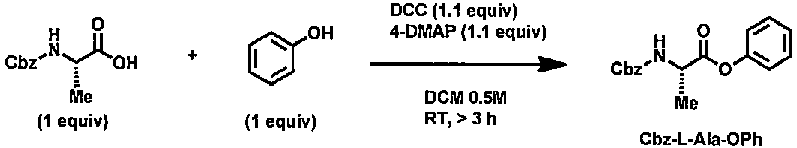

The reaction was carried out in accordance with the procedure in (Step E1-1) above except that Boc-L-Ala-OH was changed to Cbz-L-Ala-OH (CAS No.: 1142-20-7, 1.116g, 5.0mmol) to thereby afford the title compound Cbz-Ala-OPh as white solid.

### (Step E6-2) Synthesis of Cbz-L-Ala-L-Ala-Ot-Bu from Cbz-Ala-OPh

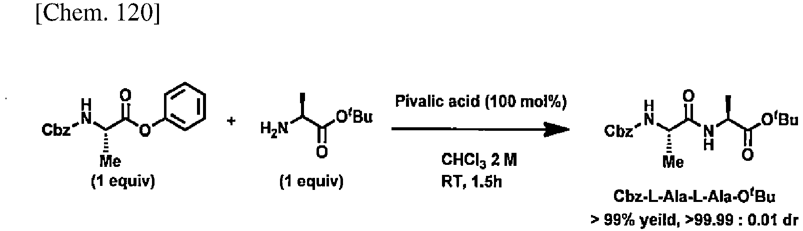

The reaction was carried out in accordance with the procedure in (Step E1-2) except that Boc-L-Ala-OPh was changed to Cbz-L-Ala-OPh prepared in (Step E6-1) (299.3mg, 1.0mmol) and the reaction time was changed from 0.5 hours to 1.5 hours to thereby afford the title compound Cbz-L-Ala-L-Ala-Ot-Bu as white solid. Yield: >99%, dr >99:1.

### ^{∗}Example E7: Synthesis of Cbz-L-Ala-L-Ala-Ot-Bu via Cbz-Ala-O(4-BrPh)

### (Step E7-1) Synthesis of Cbz-Ala-bromophenol

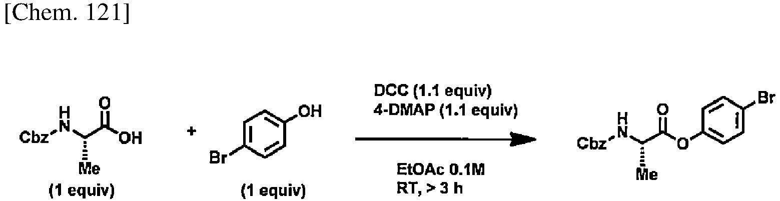

The reaction was carried out in accordance with the procedure in (Step E1-1) above except that Boc-L-Ala-OH was changed to Cbz-L-Ala-OH (CAS No.: 1142-20-7, 1.116g, 5.0mmol), Ph-OH was changed to 4-bromophenol (1.038g, 6.0mmol), and the dried DCM was changed to dried EtOAc (50mL, 0.1M) to thereby afford the title compound Cbz-Ala-O(4-BrPh) as white solid.

### (Step E7-2) Synthesis of Cbz-L-Ala-L-Ala-Ot-Bu from Cbz-Ala-O(4-BrPh)

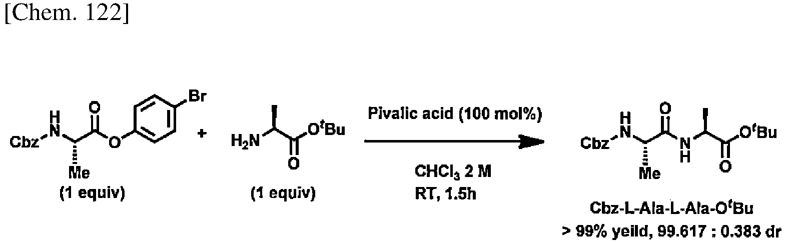

The reaction was carried out in accordance with the procedure in (step E1-2) above except that Boc-L-Ala-OPh was changed to Cbz-Ala-O(4-BrPh) prepared in (Step E7-1) (378.2mg, 1.0mmol) and the reaction time was changed from 0.5 hours to 1.5 hours to thereby afford the title compound Cbz-L-Ala-L-Ala-Ot-Bu as white solid. Yield: >99%, dr >99:1.

### ^{∗}Example E8: Synthesis of Cbz-L-Ala-L-Ala-Ot-Bu via Cbz-Ala-O(2-MePh)

### (Step E8-1) Synthesis of Cbz-Ala-O(2-MePh)

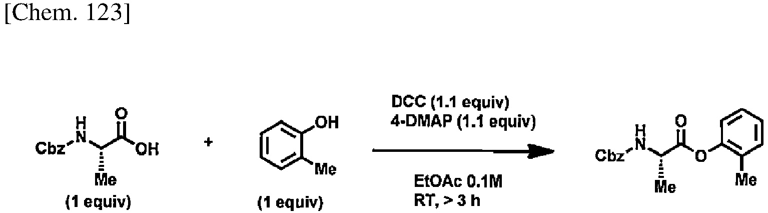

The reaction was carried out in accordance with the procedure in (Step E1-1) above except that Boc-L-Ala-OH was changed to Cbz-L-Ala-OH (CAS No.: 1142-20-7, 1.116g, 5.0mmol), Ph-OH was changed to 2-methylphenol (648.8mg, 6.0mmol), and dried DCM was changed to dried EtOAc (50mL, 0.1M) to thereby afford the title compound Cbz-Ala-O(2-MePh) as white solid.

### (Step E8-2) Synthesis of Cbz-L-Ala-L-Ala-Ot-Bu from Cbz-Ala-O(2-MePh)

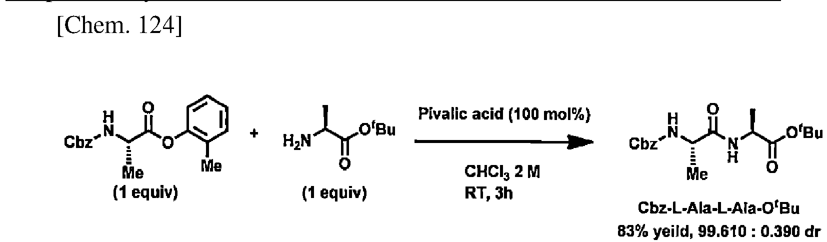

The reaction was carried out in accordance with the procedure in (Step E1-2) above except that Boc-L-Ala-OPh was changed to Cbz-Ala-O(2-MePh) prepared in (step E8-1) (313.4mg, 1.0mmol), and the reaction time was changed from 0.5 hours to 1.5 hours to thereby afford the title compound Cbz-L-Ala-L-Ala-Ot-Bu as white solid. Yield: 83%, dr >99:1.

### ^{∗}Example E9: Synthesis of Fmoc-L-Ala-L-Ala-Ot-Bu via Fmoc-Ala-OPh

### (Step E9-1) Synthesis of Fmoc-Ala-OPh

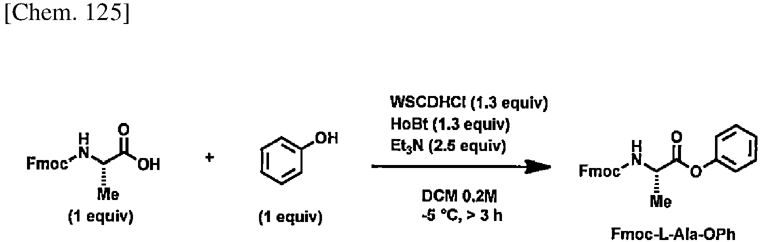

Under argon atmosphere, in a glove box, a heat-dried 5.0-mL screw-cap vial containing a stirrer (samarium-cobalt) was charged with Fmoc-L-Ala-OH (CAS No.: 35661-39-3, 1.556g, 5.0mmol) and Ph-OH (470mg, 5.0mmol). The mixture was then combined with 1-ethyl-3-(3-dimethylaminopropyl)carbodiimide hydrochloride (watersoluble carbodiimide hydrochloride, WSCDHCl) (1.245g, 6.5mmol) and 1-hydroxy benzotriazole (HoBt) (859mg, 6.5mmol), and was dissolved into dried DCM (25mL, 0.2M). Triethyl amine (Et3N) (1.7mL, 12.5mmol) was dropped into the resulting solution. The mixture was stirred vigorously under argon atmosphere at -5°C for 3 hours. The reaction was monitored by TLC analysis, and upon completion of the reaction, the reaction mixture was diluted with DCM. The reaction mixture was then purified by flash column chromatography (using 0 to 40% AcOEt in hexane as mobile phase) to thereby afford the title compound Fmoc-Ala-OPh as white solid.

### (Step E9-2) Synthesis of Fmoc-L-Ala-L-Ala-Ot-Bu from Fmoc-Ala-OPh

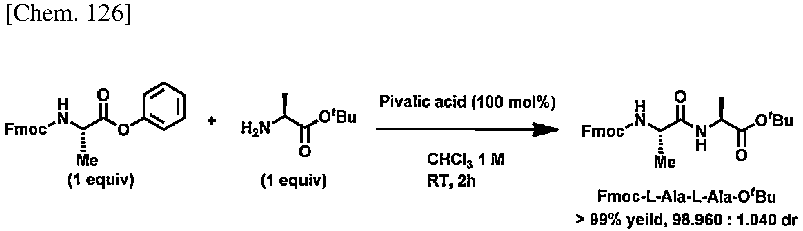

The reaction was carried out in accordance with the procedure mentioned above except that Boc-L-Ala-OPh was changed to Fmoc-Ala-OPh prepared in (step E9-1) (387.4mg, 1.0mmol), the amount of CHCl₃ used was changed from 1.5mL to 3.0mL, and the reaction time was changed from 0.5 hours to 2 hours to thereby afford the title compound Fmoc-L-Ala-L-Ala-Ot-Bu as white solid. Yield: >99%, dr: 99:1.

### ^{∗}Example E10: Synthesis of Boc-L-Ala-L-Ala-L-Ala-Ot-Bu (tripeptide) via Boc-L-Ala-OPh

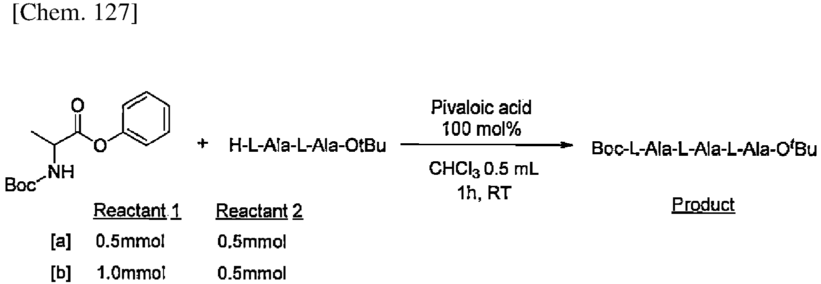

Under argon atmosphere, in a glove box, a heat-dried 5.0-mL screw-cap vial containing a stirrer (samarium-cobalt) was charged with Boc-L-Ala-OPh as Reactant Compound 1 and L-Ala-L-Ala-Ot-Bu as Reactant Compound 2 at ratios satisfying any of Condition [a] or [b] below. In addition, pivalic acid (51.1mg, 0.5mmol) was added as the Brønsted acid, and the mixture was dissolved into dried CHCl₃ (0.5mL, 1M). The resulting mixture was stirred vigorously under nitrogen atmosphere at room temperatures for 0.5 hours, and the reaction mixture was diluted with CHCl₃ (3.0 mL) and pipetted into a transferred to a silica gel column, and the used vial and pipette were washed with CHCl₃ (12.0 mL). The used vial and pipette were washed with CHCl₃ (12.0 mL). The reaction mixture was then purified by flash column chromatography (using 0 to 100%AcOEt in hexane as mobile phase) to thereby afford the title compound Boc-L-Ala-L-Ala-L-Ala-Ot-Bu as white solid.
^{∗}Condition [a]: Reactant Compound 1 = 0.5 mmol, Reactant Compound 2 = 0.5mmol. The yield was 26% and dr was 99:1 under this condition.
^{∗}Condition [b]: Reactant Compound 1 = 1.0mmol, Reactant Compound 2 = 0.5mmol. The yield was 40% and dr was 98:2 under this condition.

## Claims

1. A catalyst for amide reaction between a carboxylic acid ester group and an amino group, said catalyst comprising a Brønsted acid.

2. The catalyst according to claim 1, wherein the Brønsted acid is selected from the group consisting of compounds with pKa values of 6.0 or less.

3. The catalyst according to claim 1 or 2, wherein the Brønsted acid is selected from the group consisting of aliphatic or aromatic hydrocarbons having one or more carboxyl groups.

4. A method of producing, from a compound represented by general formula (1-1) and a compound represented by general formula (1-2), an amide compound represented by general formula (1-3), comprising:
causing amide reaction between the compound represented by general formula (1-1) and the compound represented by general formula (1-2) in the presence of a Brønsted acid according to any one of claims 1 to 3. 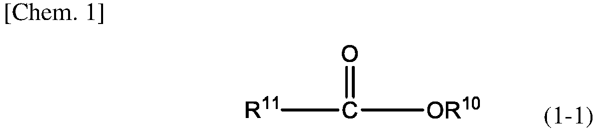
In general formula (1-1),
R¹⁰ represents a monovalent hydrocarbon group or heterocyclic group that may have one or more substituents, and
R¹¹ represents a monovalent hydrocarbon group or heterocyclic group that may have one or more substituents or a monovalent group formed by linking, optionally via a linking group, two or more multivalent hydrocarbon and/or heterocyclic groups that each may have one or more substituents. 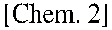 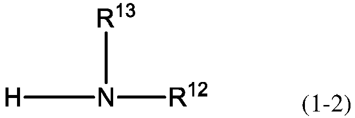
In general formula (1-2),
R¹² represents a monovalent hydrocarbon group or heterocyclic group that may have one or more substituents or a monovalent group formed by linking, optionally via a linking group, two or more multivalent hydrocarbon and/or heterocyclic groups that each may have one or more substituents, and
R¹³ represents a hydrogen atom, carboxyl group, or hydroxyl group, or a monovalent hydrocarbon group or heterocyclic group that may have one or more substituents and may be bound to the nitrogen atom via a linking group, or
R¹² and R¹³ may be bound to each other to form, together with the nitrogen atom to which R¹² and R¹³ bind, a hetero ring that may have one or more substituents. 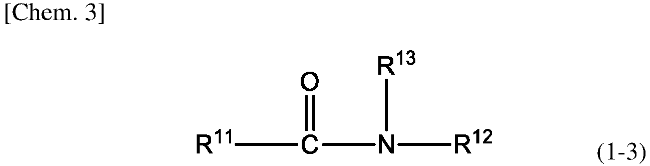
In general formula (1-3), each symbol represents the same definition as that of the same symbol in general formulae (1-1) and (1-2) above.

5. A method of producing, from a compound represented by general formula (2-1), an amide compound represented by general formula (2-2), comprising:
causing intramolecular amide reaction in the compound represented by general formula (2-1) in the presence of a Brønsted acid according to any one of claims 1 to 3. 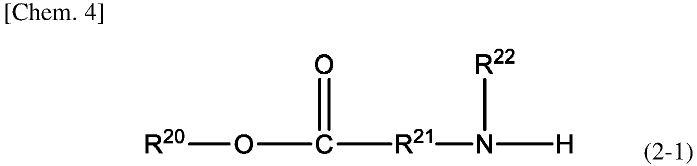
In general formula (2-1),
R²⁰ represents a monovalent hydrocarbon group or heterocyclic group that may have one or more substituents, and
R²¹ represents a divalent hydrocarbon group or heterocyclic group that may have one or more substituents or a divalent group formed by linking, optionally via a linking group, two or more multivalent hydrocarbon and/or heterocyclic groups that each may have one or more substituents, and
R²² represents a hydrogen atom, carboxyl group, or hydroxyl group, or a monovalent hydrocarbon group or heterocyclic group that may have one or more substituents and may be bound to the nitrogen atom via a linking group, or
R²¹ and R²² may be bound to each other to form, together with the nitrogen atom to which R²¹ and R²² bind, a hetero ring that may have one or more substituents. 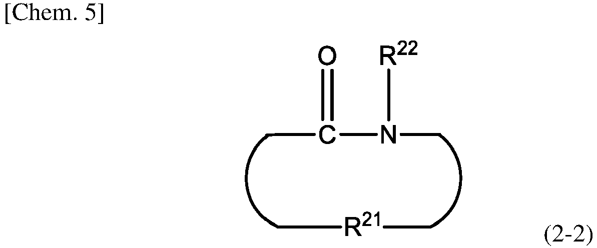
In general formula (2-2), each symbol represents the same definition as that of the same symbol in general formula (2-1) above.

6. A method of producing, from a compound represented by general formula (3-1) and a compound represented by general formula (3-2), an amide compound represented by general formula (3-3), comprising:
causing amide reaction between the compound represented by general formula (3-1) and the compound represented by general formula (3-2) in the presence of a Brønsted acid according to any one of claims 1 to 3. 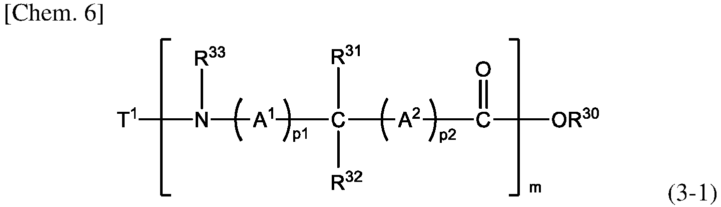
In general formula (3-1),
R³⁰ represents a monovalent hydrocarbon group or heterocyclic group that may have one or more substituents, and
R³¹ and R³², independently of each other, represent a hydrogen atom, halogen atom, hydroxyl group, carboxyl group, nitro group, cyano group, thiol group, or, a monovalent hydrocarbon group or heterocyclic group that may have one or more substituents and may be bound to the carbon atom via a linking group, and
R³³ represents a hydrogen atom, carboxyl group, hydroxyl group, or, a monovalent hydrocarbon group or heterocyclic group that may have one or more substituents and may be bound to the nitrogen atom via a linking group, or
R³¹ and R³³ may be bound to each other to form, together with the carbon atom to which R³¹ binds and the nitrogen atom to which R³³ binds, a hetero ring that may have one or more substituents,
A¹ and A², independently of each other, represent a divalent aliphatic hydrocarbon group that may have one or more substituents having 1 to 3 carbon atoms,
T¹ represents a hydrogen atom or a monovalent substituent,
p1 and p2, independently of each other, represent an integer of 0 or 1, and
m represents an integer of equal to or greater than 1 corresponding to the number of the structure units parenthesized with [ ], provided that when m is equal to or greater than 2, then the two or more structure units in [ ] may be either identical to each other or different from each other. 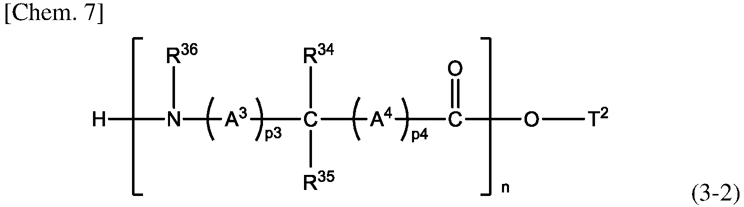
In general formula (3-2),
R³⁴ and R³⁵, independently of each other, represent a hydrogen atom, halogen atom, hydroxyl group, carboxyl group, nitro group, cyano group, thiol group, or, a monovalent hydrocarbon group or heterocyclic group that may have one or more substituents and may be bound to the carbon atom via a linking group,
R³⁶ represents a hydrogen atom, carboxyl group, hydroxyl group, or, a monovalent hydrocarbon group or heterocyclic group that may have one or more substituents and may be bound to the nitrogen atom via a linking group,
R³⁴ and R³⁶ may be bound to each other to form, together with the carbon atom to which R³⁴ binds and the nitrogen atom to which R³⁶ binds, a hetero ring that may have one or more substituents,
A³ and A⁴, independently of each other, represent a divalent aliphatic hydrocarbon group that may have one or more substituents having 1 to 3 carbon atoms,
T² represents a hydrogen atom or a monovalent substituent,
p3 and p4, independently of each other, represent an integer of 0 or 1, and
n represents an integer of equal to or greater than 1 corresponding to the number of the structure units parenthesized with [ ], provided that when n is equal to or greater than 2, then the two or more structure units in [ ] may be either identical to each other or different from each other. 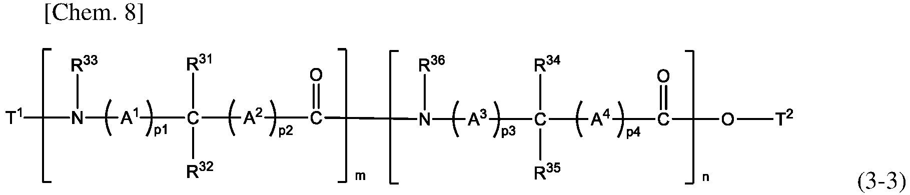
In general formula (3-3), each symbol represents the same definition as that of the same symbol in general formulae (3-1) and (3-2) above.

7. The method according to any one of claims 4 to 6, wherein R¹⁰ in general formula (1-1), R²⁰ in general formula (2-1), or R³⁰ in general formula (3-1) is a monovalent aromatic group or heterocyclic group which may have one or more substituents.

8. The method according to any one of claims 3 to 7, wherein the reaction is carried out as a batch reaction or a flow reaction.
